# EUROPEAN PATENT APPLICATION

(11) **EP 3 970 686 A1**
(43) Date of publication of application: **23.03.2022**
(21) Application number: 21157455.3
(22) Date of filing: 16.02.2021
(51) Int. Cl.: A61H 23/02

(54) **DEVICES AND METHODS FOR USING MECHANICAL AFFECTIVE TOUCH THERAPY TO REDUCE STRESS, ANXIETY AND DEPRESSION**

(30) Priority: 20.09.2020 US 202017026268
(71) Applicant: APEX NEURO HOLDINGS, INC, Brooklyn, NY 11201 (US)
(72) Inventor: Garikapati, Durga Sahithi, Brooklyn, NY 11201 (US); Hagberg, Sean, Brooklyn, NY 11201 (US); Kress, Francois, Brooklyn, NY 11201 (US)
(74) Representative: AWA Sweden AB

(57) **Abstract**

Methods and devices that reduce stress, anxiety and/or depression in a human using mechanical affective touch therapy is provided. In on embodiment, the method comprises (1) generating mechanical vibrations using a sinusoidal waveform and a mechanical transducer of a transcutaneous mechanical stimulation device in response to an applied electronic drive signal, (2) controlling the mechanical vibrations of the electronic drive signal by a controller board so that the mechanical vibrations have a frequency of less than 20 Hz; and (3) delivering the mechanical vibrations to the body of the human via the mechanical stimulation device, thereby providing the human with transcutaneous mechanical stimulation that reduces the human's anxiety, stress and/or depression.

## Description

### FIELD OF THE INVENTION

The present invention relates generally to wearable devices and associated methods that provide a variety of health benefits to humans. In particular, certain embodiments of the devices and associated methods disclosed herein, show a significant reduction in stress, anxiety and depression in humans using the mechanically affective touch therapy devices and associated methods disclosed herein.

### BACKGROUND

Systematic testing and associated survey was performed on various waveforms, frequencies and physical locations on the body using different mechanical transducers (actuators more specifically, as actuators use electrical energy to generate mechanical energy) and different signal generators to assess their effects in human subjects. The first set of testing and associated survey focused on mechanical stimulation of cranial nerves. Various parameters, including heart rate, blood pressure, respiration and mood were monitored and tested for their affect in humans. After testing about 56 waveforms, 27 different piezo device combinations and various different anatomical placements in over 1600 human subjects, the data indicated that the devices and methods tested showed no reliable effects on the cranial nerves in the physiological outcomes being collected, although anecdotally, longer term users of the same devices and methods reported significant benefits.

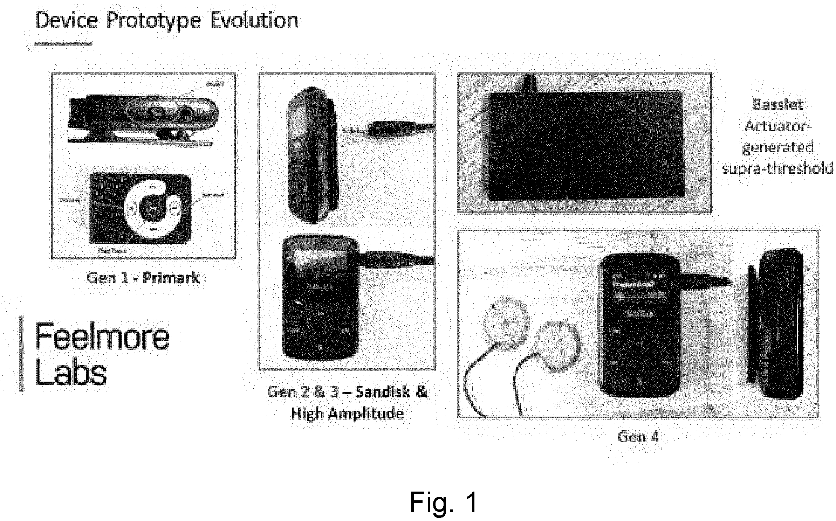

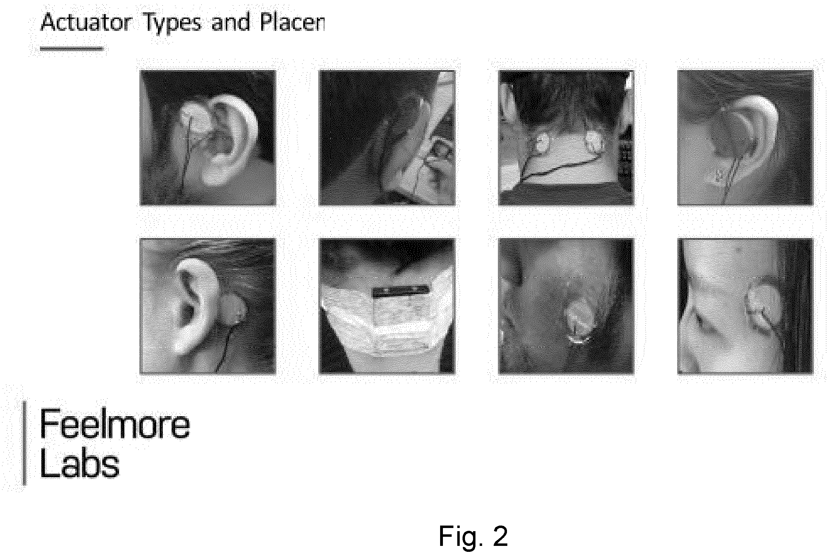

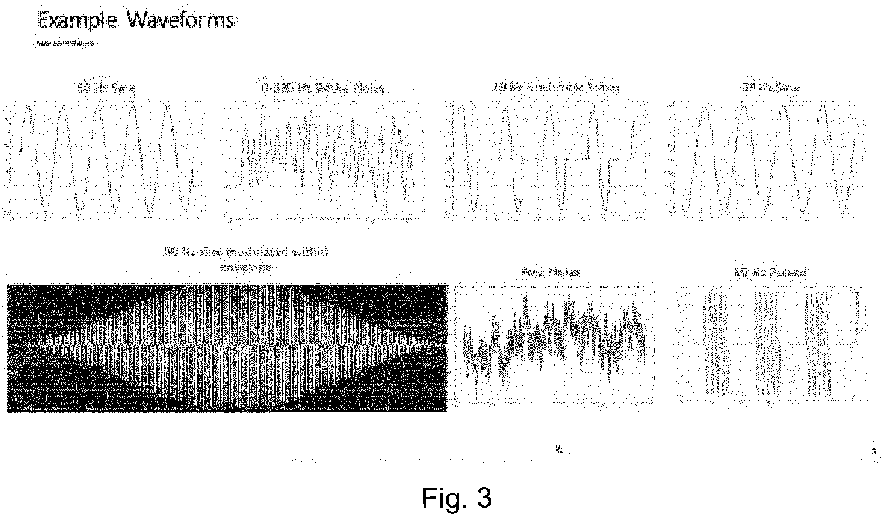

**FIG. 1** shows images of some of the devices that were used and their evolution. **FIG. 2** shows some of the actuators that were used and some of their placements on the human body for the various experiments performed. **FIG. 3** shows images of some of the waveforms that were tested with the various actuators on humans.

EEG equipment was then used to ascertain the effects of the mechanical stimulation of brain state. Different waveforms were systematically tested for efficacy. The results of our extensive testing suggested that the narrow range of 1-20 Hz wave forms provided positive effects on brain state by increasing alpha power and therefore providing greater relaxation and focus. With certain characteristics of the waveform that provides benefits established, we began systematically testing various types of delivery systems. These included multiple types of actuators from at least three 'families' of actuators, 1) Piezo-electrical (more properly reverse piezo-electrical), 2) LRAs (linear resonant actuators) and 3) ERM (electrical rotating mass. After determining which actuators provided the most desirable benefits and advantages in humans, we then systematically investigated power, as defined by voltage, to assess the effects of changes in power on outcomes after providing the most beneficial waveforms using the most beneficial actuators in humans. By systematically testing the various factors, we determined that specific signal ranges were more likely to produce a beneficial or signature response from the subject EEG, which was an increase in alpha power and a decrease in other bands (beta, theta, gamma).

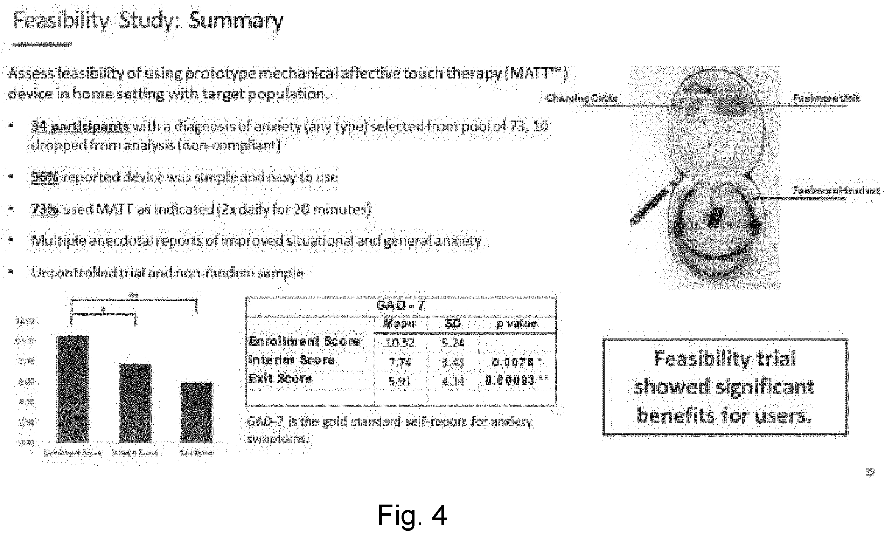

The waveforms demonstrating the most efficacy in humans were then delivered in two separate research trials, Trial A and Trial B, that were conducted over 30 days in subjects diagnosed with an anxiety disorder and potentially co-morbid psychiatric diagnoses (such as Depression). In both cases, all human subjects were screened for anxiety symptoms in addition to a formal diagnosis of anxiety took a battery of self-reports. In Trial B, the human subjects had both EEG and MRI before and immediately after first use of the device and then after 30 days of use to further elaborate any benefits of the device and associated methods provided to the human. **FIGS. 4** amd **5** shows the summary results of Trial A and follow-up summary respectively. In the feasibility study follow-up, 17 participants of the Trial A feasibility study were contacted 5 months after the conclusion of the Trial A feasibility study. Sixteen out of seventeen participants contacted reported a decrease in anxiety symptoms during the trial. Eight participants experienced a decrease in their anxiety for at least one week after the conclusion of the study. Of those eight participants, four participants had a decrease in their anxiety for over one month after discontinuation. Three participants reported having an on-going reduction in their anxiety symptoms, and increased stress management abilities. Six participants felt the device was more effective for coping with their symptoms than their current methods, which encouraged us to proceed with the more comprehensive study and inventions described herein.

Accordingly, for all the reasons mentioned above, there is a need for improved systems, methods, and devices that reduce stress, anxiety and depression with little to no side effects and a well-established and robust safety profile. The inventions of the present disclosure provide these and other related benefits and advantages.

### SUMMARY OF THE INVENTION

Presented herein are methods, and devices that reduce stress, anxiety and depression using mechanical affective touch therapy (MATT). In certain embodiments, the approaches described herein utilize a stimulation device (e.g., a wearable or applied device) for generation and delivery of the mechanical vibrational waves.

As described herein, the delivered vibrational waves can be tailored based on particular targets (e.g., nerves, mechanoreceptors, vascular targets, tissue regions) to stimulate and/or to elicit particular desired responses in a subject. As described herein, in certain embodiments, the delivery of mechanical stimulation to a subject provides for treatment of anxiety.

In certain embodiments, the properties of mechanical waves generated are tailored by controlling a waveform of an electronic drive signal that is applied to mechanical transducers in order to generate a desired mechanical wave. By controlling and delivering various specific mechanical waves in this manner, the approaches described herein can be used to achieve a variety of health benefits in subjects, for example and not by way of limitation, reducing stress, depression and/or anxiety.

In at least one embodiment of this disclosure, a device for reducing anxiety, depression and/or stress in a human is provided. The device comprises (1) one or more mechanical transducers, (2) one or more batteries, (3) one or more sinusoidal waveforms and (4) one or more controller boards that control at least the one or more sinusoidal waveforms output through the mechanical transducers. The one or more mechanical transducers, batteries and controller boards are in communication with each other. The controller board controls the sinusoidal waveform output through the one or more mechanical transducers, thereby producing mechanical vibrations for a human. The device is adapted to provide mechanical vibrations in proximity to the temporal bone of the human's head.

In at least one aspect of at least one embodiment of this disclosure, the frequency of the one or more waveform is less than 20 Hz.

In another aspect of at least one embodiment of this disclosure, the frequency of the one or more waveforms is approximately 10 Hz.

In another aspect of at least one embodiment of this disclosure, the one or more waveforms are isochronic.

In another aspect of at least one embodiment of this disclosure, the device delivers mechanical vibrations in proximity to the temporal bone for at least 10 minutes per day.

In another aspect of at least one embodiment of this disclosure, the device delivers mechanical vibrations in proximity to the temporal bone at least one time per day for a period of at least 4 weeks.

In at least one other embodiment of the present disclosure, a method of reducing anxiety, stress or depression in a human is provided. The method comprises (1) generating mechanical vibrations using a sinusoidal waveform and a mechanical transducer of a transcutaneous mechanical stimulation device in response to an applied electronic drive signal, (2) controlling the mechanical vibrations of the electronic drive signal by a controller board so that the mechanical vibrations have a frequency of less than 20 Hz; and (3) delivering the mechanical vibrations to the body of the human via the mechanical stimulation device, thereby providing the human with transcutaneous mechanical stimulation that reduces the human's anxiety, stress and/or depression.

In at least another aspect of at least one embodiment of the invention, the mechanical vibrations are provided to the C-tactile afferents of the human's head.

In at least another aspect of at least one embodiment of the invention, the device delivers mechanical vibrations to the humans head area at least 20 minutes per day.

In at least another aspect of at least one embodiment of the invention, the device delivers mechanical vibrations to the humans head area at least 2 times per day.

In another embodiment of the present invention, a device for reducing depression in a human is provided. The device comprises one or more mechanical transducers, one or more batteries and one or more controller boards, where the one or more mechanical transducers, the one or more batteries and the one or more controller boards are in communication and when the device's mechanical transducers provide mechanical vibrations near the human's head, the human's depression is reduced.

In yet another embodiment of the present invention, a device for reducing stress in a human is provided. The device comprises one or more mechanical transducers, one or more batteries, and one or more controller boards, where the one or more mechanical transducers, the one or more batteries and the one or more controller boards are in communication and when the device's mechanical transducers provide mechanical vibrations near the human's head, the human's stress is reduced.

In yet another embodiment of the present invention, a method of reducing anxiety, stress or depression in a human is provided. The method comprises (1) generating mechanical vibrations using a mechanical transducer of a transcutaneous mechanical stimulation device in response to an applied electronic drive signal, (2) controlling the mechanical vibrations of the electronic drive signal by a controller board so that the mechanical vibrations have a frequency of less than 20 Hz and (3) delivering the mechanical vibrations to the body of the human via the mechanical stimulation device, thereby providing the human with transcutaneous mechanical stimulation that reduces the human's anxiety, stress and/or depression.

In yet another aspect of at least one embodiment, the invention is directed to a transcutaneous neuromodulation device [e.g., a wearable device; e.g., a non-invasive device (e.g., not comprising any components that penetrate skin)] for treating anxiety and/or an anxiety related disorder in a subject by promoting nerve stimulation through mechanical vibration, comprising: one or more mechanical transducers, a battery, and one or more controller boards, wherein the one or more mechanical transducers, the battery and the one or more controller boards are in communication (e.g., through one or more connectors; e.g., wirelessly), and wherein the controller board controls waveform output through the one or more mechanical transducers, thereby producing mechanical vibration.

In at least some embodiment of the present invention, the device promotes stimulation (e.g., wherein the waveform is selected to promote stimulation) of one or more nerves [e.g., a vagus nerve; e.g., a trigeminal nerve; e.g., peripheral nerves; e.g., a greater auricular nerve; e.g., a lesser occipital nerve; e.g., one or more cranial nerves (e.g., cranial nerve VII; e.g., cranial nerve IX; e.g., cranial nerve XI; e.g., cranial nerve XII)].

In certain embodiments, the one or more nerves comprises a vagus nerve and/or a trigeminal nerve.

In certain embodiments, the one or more nerves comprises a C-tactile afferent.

In certain embodiments, the device promotes stimulation of (e.g., wherein the waveform is selected to promote stimulation of) one or more mechanoreceptors and/or cutaneous sensory receptors in the skin (e.g., to stimulate an afferent sensory pathway and use properties of receptive fields to propagate stimulation through tissue and bone). In certain embodiments, the one or more mechanoreceptors and/or cutaneous sensory receptors comprise Piezo2 protein and/or Merkel cells.

In certain embodiments, the one or more controller boards modulate the waveform output to introduce particular signals that include active or inactive pulse durations and frequencies configured to accommodate particular mechanoreceptor recovery periods, adaptation times, inactivation times, sensitization and desensitization times, or latencies.

In certain embodiments, the one or more controller boards modulate the waveform output to enhance or inhibit the expression of presynaptic molecules essential for synaptic vesicle release in neurons.

In certain embodiments, the one or more controller boards modulate the waveform output to enhance or inhibit the expression of neuroactive substances that can act as fast excitatory neurotransmitters or neuromodulators.

In certain embodiments, the one or more controller boards modulates the waveform output to stimulate mechanoreceptor cell associated with Aδ-fibers and C-fibers (e.g., including C tactile fibers) in order to stimulate nociceptive, thermoceptive and other pathways modulated by these fibers.

In certain embodiments, the one or more controller boards modulate the waveform output using dynamical systems methods to produce a preferred response in neural network dynamics (e.g., via modulation of signal timing).

In certain embodiments, the one or more controller boards modulates the waveform output using dynamical systems measures to assess response signals (e.g., electronic) to detect particular network responses correlated with changes in mechanical wave properties (e.g., and modulates the waveform output to target/optimally enhance particular preferred responses).

In certain embodiments, the device comprises at least one transducer array comprising a plurality of (e.g., two or more) mechanical transducers maintained in a fixed spatial arrangement in relation to each other (e.g., in substantial proximity to each other; e.g., spaced along a straight or curved line segment) and wherein at least a portion of the one or more controller boards (e.g., a single controller board; e.g., two or more controller boards) are in communication with the mechanical transducers of the transducer array to control output of the mechanical transducers of the transducer array in relation to each other [e.g., wherein the at least a portion of the one or more controller boards synchronizes mechanical vibration produced by each mechanical transducer of the transducer array (e.g., such that each mechanical transducer begins and/or ends producing mechanical vibration at a particular delay with respect to one or more other mechanical transducers of the array; e.g., such that the mechanical transducers are sequentially triggered, one after the other; e.g., wherein the mechanical transducers are spaced along a straight or curved line segment and triggered sequentially along the line segment, such that an apparent source of mechanical vibration moves along the line segment to mimic a stroking motion)] [e.g., wherein a first portion of the mechanical transducers outputs a different frequency mechanical vibration from a second portion of the mechanical transducers of the transducer array (e.g., wherein each mechanical transducer of the transducer array outputs a different frequency mechanical vibration)].

In certain embodiments, the transducer is a linear transducer (e.g., operable to produce mechanical vibration comprising a longitudinal component (e.g., a longitudinal vibration)).

In certain embodiments, the device comprises a receiver in communication with the one or more controller boards, wherein the receiver is operable to receive a signal from and/or transmit a signal (e.g., wirelessly; e.g., via a wired connection) to a personal computing device

(e.g., a smart phone; e.g., a personal computer; e.g., a laptop computer; e.g., a tablet computer; e.g., a smartwatch; e.g., a fitness tracker; e.g., a smart charger)(e.g., to upload new waveforms and/or settings for waveforms).

In certain embodiments, the one or more controller boards is/are operable to modulate and/or select the waveform output in response to (e.g., based on) the signal received from the personal computing device by the receiver.

In certain embodiments, one or more low-amplitude sub-intervals of the isochronic wave have a duration of greater than or approximately two seconds (e.g., wherein the one or more low-amplitude sub-intervals have a duration of approximately two seconds; e.g., wherein the one or more low-amplitude sub-intervals have a duration ranging from approximately two seconds to approximately 10 seconds; e.g., wherein the one or more low amplitude sub-intervals have a duration ranging from approximately two seconds to approximately 4 seconds).

In certain embodiments, the isochronic wave comprises a carrier wave [e.g., a periodic wave having a substantially constant frequency (e.g., ranging from 0 to 20 Hz; or approximately 7 to approximately 13 Hz; e.g., a frequency range matching an alpha brain wave frequency range; e.g., approximately 10 Hz)] modulated by an envelope function having one or more low-amplitude sub-intervals [e.g., a periodic envelope function (e.g., a square wave; e.g., a 0.5 Hz square wave); e.g., the one or more low-amplitude sub-intervals having a duration of greater than or approximately equal to two seconds; e.g., the one or more low-amplitude sub-intervals having a duration of approximately two seconds].

In certain embodiments, the isochronic wave is also a transformed time-varying wave. In certain embodiments, the isochronic wave comprises a chirped wave. In certain embodiments, the waveform output comprises a transformed time-varying wave having a functional form corresponding to a carrier wave within an envelope {e.g., wherein the transformed-time varying wave is the carrier wave and is further modulated by an envelope [e.g., wherein the envelope is a sinusoidal wave; e.g., wherein the envelope has a monotonically increasing (in time) amplitude (e.g., wherein the envelope has a functional form corresponding to an increasing (in time) exponential)]; e.g., wherein the transformed time-varying wave is the envelope that modulates a carrier wave [e.g., wherein the carrier wave is a periodic wave (e.g., a sinusoidal wave; e.g., a square wave; e.g., a sawtooth wave)(e.g., having a higher frequency than the envelope)]}.

In certain embodiments, the device comprises a receiver in communication with the one or more controller boards, wherein the receiver is operable to receive a signal from and/or transmit a signal to a monitoring device (e.g., directly from and/or to the monitoring device; e.g., via one or more intermediate server(s) and/or computing device(s))(e.g., a wearable monitoring device; e.g., a personal computing device; e.g., a fitness tracker;. e.g., a heart-rate monitor; e.g., an electrocardiograph (EKG) monitor; e.g., an electroencephalography (EEG) monitor; e.g., an accelerometer; e.g., a blood-pressure monitor; e.g., a galvanic skin response (GSR) monitor) and wherein the one or more controller boards is/are operable to modulate and/or select the waveform output in response to (e.g., based on) the signal from the wearable monitoring device received by the receiver.

In certain embodiments, the device is operable to record usage data (e.g., parameters such as a record of when the device was used, duration of use, etc.) and/or one or more biofeedback signals for a human subject [e.g., wherein the device comprises one or more sensors, each operable to measure and record one or more biofeedback signals (e.g., a galvanic skin response (GSR) sensor; e.g., a heart-rate monitor; e.g., an accelerometer)] [e.g., wherein the device is operable to store the recorded usage data and/or biofeedback signals for further processing and/or transmission to an external computing device, e.g., for computation (e.g., using a machine learning algorithm that receives the one or more biofeedback signals as input, along with, optionally, user reported information) and display of one or more performance metrics (e.g., a stress index) to a subject using the device].

In certain embodiments, the one or more controller boards is/are operable to automatically modulate and/or select the waveform output in response to (e.g., based on) the recorded usage data and/or biofeedback signals (e.g., using a machine learning algorithm that receives the one or more biofeedback signals as input, along with, optionally, user reported information, to optimize the waveform output).

In certain embodiments, a level [e.g., amplitude (e.g., a force; e.g., a displacement)] of at least a portion of the mechanical vibration is based on activation thresholds of one or more target cells and/or proteins (e.g., mechanoreceptors (e.g., C tactile afferents); e.g., nerves; e.g., sensory thresholds corresponding to a level of tactile sensation) [e.g., wherein the one or more controller boards modulate the waveform output based on sub-activation thresholds (e.g., accounting for the response of the mechanical transducers)].

In certain embodiments, an amplitude of the mechanical vibration corresponds to a displacement ranging from 1 micron to 10 millimeters (e.g., approximately 25 microns and in at least one embodiment 0.01 mm)(e.g., wherein the amplitude is adjustable over the displacement ranging from 1 micron to 10 millimeters) [e.g., wherein the amplitude corresponds to a force of approximately 0.4N][e.g., thereby matching the amplitude to activation thresholds of C tactile afferents].

In certain embodiments, the isochronic wave comprises one or more components (e.g., additive noise; e.g., stochastic resonance signals) that, when transduced by the transducer to produce the mechanical wave, correspond to sub-threshold signals that are below an activation threshold of one or more target cells and/or proteins (e.g., below a level of tactile sensation).

In certain embodiments, the isochronic wave comprises one or more components (e.g., additive noise; e.g., stochastic resonance signals) that, when transduced by the transducer to produce the mechanical wave, correspond to supra-threshold signals that are above an activation threshold of one or more target cells and/or proteins (e.g., above a level of tactile sensation).

In another aspect, the invention is directed to a transcutaneous neuromodulation device [e.g., a wearable device; e.g., a non-invasive device (e.g., not comprising any components that penetrate skin)] for treating anxiety and/or an anxiety related disorder in a human subject by promoting nerve stimulation through mechanical vibration, comprising: one or more mechanical transducers, a battery, and one or more controller boards, wherein the one or more mechanical transducers, the battery and the one or more controller boards are in communication (e.g., through one or more connectors; e.g., wirelessly), and wherein the one or more controller boards control waveform output through the one or more mechanical transducers, and the one or more mechanical transducers transcutaneously stimulate one or more nerves of a human subject and wherein the waveform output comprises an isochronic wave.

In another aspect, the invention is directed to a transcutaneous stimulation device [e.g., a wearable device; e.g., a non-invasive device (e.g., not comprising any components that penetrate skin)] for treating anxiety and/or an anxiety related disorder in a human subject by promoting mechanoreceptor stimulation through mechanical vibration, comprising: one or more mechanical transducers, a battery, and one or more controller boards, wherein the one or more mechanical transducers, the battery and the one or more controller boards are in communication (e.g., through one or more connectors; e.g., wirelessly), and wherein the one or more controller boards control waveform output through the transducer, and the one or more mechanical transducers transcutaneously stimulate one or more mechanoreceptors of a human subject and wherein the waveform output comprises an isochronic wave.

In another aspect, the invention is directed to a method of treating anxiety and/or an anxiety related disorder in a subject by providing transcutaneous mechanical stimulation (e.g., non-invasive mechanical stimulation) to the subject via a stimulation device (e.g., a wearable device), the method comprising: generating a mechanical wave by a mechanical transducer of the stimulation device in response to an applied electronic drive signal; controlling a waveform of the electronic drive signal by a controller board (e.g., a controller board of the stimulation device; e.g., a remote controller board), wherein the waveform comprises an isochronic wave; and delivering the mechanical wave to a body location of the subject via the stimulation device, thereby providing the transcutaneous mechanical stimulation to the subject.

In certain embodiments, the mechanical wave promotes stimulation (e.g., wherein the waveform is selected to promote stimulation) of one or more nerves [e.g., a vagus nerve; e.g., a trigeminal nerve; e.g., peripheral nerves; e.g., a greater auricular nerve; e.g., a lesser occipital nerve; e.g., one or more cranial nerves (e.g., cranial nerve VII; e.g., cranial nerve IX; e.g., cranial nerve XI; e.g., cranial nerve XII)]. In certain embodiments, the one or more nerves comprises a vagus nerve and/or a trigeminal nerve. In certain embodiments, the one or more nerves comprises a C-tactile afferent.

In certain embodiments, the mechanical wave promotes stimulation of (e.g., wherein the waveform is selected to promote stimulation of) one or more mechanoreceptors and/or cutaneous sensory receptors in the skin (e.g., to stimulate an afferent sensory pathway and use properties of receptive fields to propagate stimulation through tissue and bone). In certain embodiments, the one or more mechanoreceptors and/or cutaneous sensory receptors comprise Piezo2 protein and/or Merkel cells.

In certain embodiments, the controlling the waveform of the electronic drive signal comprises modulating the waveform to introduce particular signals that include active or inactive pulse durations and frequencies configured to accommodate particular mechanoreceptor recovery periods, adaptation times, inactivation times, sensitization and desensitization times, or latencies.

In certain embodiments, the controlling the waveform of the electronic drive signal comprises modulating the waveform to enhance or inhibit the expression of presynaptic molecules essential for synaptic vesicle release in neurons.

In certain embodiments, the controlling the waveform of the electronic drive signal comprises modulating the waveform to enhance or inhibit the expression of neuroactive substances that can act as fast excitatory neurotransmitters or neuromodulators.

In certain embodiments, the controlling the waveform of the electronic drive signal comprises modulating the waveform to stimulate mechanoreceptor cells associated with Aδ-fibers and C-fibers (e.g., including C tactile fibers) in order to stimulate nociceptive, thermoceptive, interoceptive and/or other pathways modulated by these fibers.

In certain embodiments, the controlling the waveform of the electronic drive signal comprises modulating the waveform using dynamical systems methods to produce a preferred response in neural network dynamics (e.g., via modulation of signal timing).

In certain embodiments, the controlling the waveform of the electronic drive signal comprises modulating the waveform using dynamical systems measures to assess response signals (e.g., electronic) to detect particular network responses correlated with changes in mechanical wave properties (e.g., and modulates the waveform output to target/optimally enhance particular preferred responses).

In certain embodiments, the delivering the mechanical wave to the body location comprises contacting the mechanical transducer to a surface (e.g., skin) of the subject at the body location.

In certain embodiments, the mechanical transducer is a member of a transducer array comprising a plurality of (e.g., two or more) mechanical transducers maintained in a fixed spatial arrangement in relation to each other (e.g., in substantial proximity to each other; e.g., spaced along a straight or curved line segment) and wherein the controller board controls output of the mechanical transducer in relation to other mechanical transducers of the array [e.g., so as to synchronize mechanical vibration produced by each mechanical transducer of the transducer array (e.g., such that each mechanical transducer begins and/or ends producing mechanical vibration at a particular delay with respect to one or more other mechanical transducers of the array; e.g., such that the mechanical transducers are sequentially triggered, one after the other; e.g., wherein the mechanical transducers are spaced along a straight or curved line segment and triggered sequentially along the line segment, such that an apparent source of mechanical vibration moves along the line segment to mimic a stroking motion)] [e.g., wherein a first portion of the mechanical transducers outputs a different frequency mechanical vibration from a second portion of the mechanical transducers of the transducer array (e.g., wherein each mechanical transducer of the transducer array outputs a different frequency mechanical vibration)].

In certain embodiments, the transducer is a linear transducer (e.g., operable to produce mechanical vibration comprising a longitudinal component (e.g., a longitudinal vibration)).

In certain embodiments, the mechanical transducer is incorporated into a headphone (e.g., an in-ear headphone; e.g., an over-the-ear headphone).

In certain embodiments, the controlling the waveform of the electronic drive signal comprises receiving (e.g., by a receiver in communication with the controller board) a signal from a personal computing device (e.g., a smart phone; e.g., a personal computer; e.g., a laptop computer; e.g., a tablet computer; e.g., a smartwatch; e.g., a fitness tracker; e.g., a smart charger)(e.g., to upload new waveforms and/or settings for waveforms).

In certain embodiments, the controlling the waveform of the electronic drive signal comprises modulating and/or selecting the waveform in response to (e.g., based on) the signal received from the personal computing device by the receiver.

In certain embodiments, the delivering the mechanical wave to the body location is performed in a non-invasive fashion (e.g., without penetrating skin of the subject).

In certain embodiments, the method comprising providing, by a secondary stimulation device, one or more external stimulus/stimuli (e.g., visual stimulus; e.g., acoustic stimulus; e.g., limbic priming; e.g., a secondary tactile signal).

In certain embodiments, the isochronic wave comprises a frequency component ranging from 5 to 15 Hz (e.g., ranging from approximately 7 to approximately 13 Hz; e.g., a frequency range matching an alpha brain wave frequency range; e.g., approximately 10 Hz).

In certain embodiments, the isochronic wave comprises a frequency component ranging from 0 to 49 Hz (e.g., from 18 to 48 Hz; e.g., from 15 to 40 Hz; e.g. from 8 to 14 Hz).

In certain embodiments, one or more low-amplitude sub-intervals of the isochronic wave have a duration of greater than or approximately two seconds (e.g., wherein the one or more low-amplitude sub-intervals have a duration of approximately two seconds; e.g., wherein the one or more low-amplitude sub-intervals have a duration ranging from approximately two seconds to approximately 10 seconds; e.g., wherein the one or more low amplitude sub-intervals have a duration ranging from approximately two seconds to approximately 4 seconds).

In certain embodiments, the isochronic wave comprises a carrier wave [e.g., a periodic wave having a substantially constant frequency (e.g., ranging from 5 to 15 Hz; e.g., ranging from approximately 7 to approximately 13 Hz; e.g., a frequency range matching an alpha brain wave frequency range; e.g., approximately 10 Hz)] modulated by an envelope function having one or more low-amplitude sub-intervals [e.g., a periodic envelope function (e.g., a square wave; e.g., a 0.5 Hz square wave); e.g., the one or more low-amplitude sub-intervals having a duration of greater than or approximately equal to two seconds; e.g., the one or more low-amplitude sub-intervals having a duration of approximately two seconds].

In certain embodiments, the isochronic wave is also a transformed time-varying wave. In certain embodiments, the isochronic wave comprises a chirped wave. In certain embodiments, the waveform of the electronic drive signal comprises a transformed time-varying wave having a functional form corresponding to a carrier wave within an envelope {e.g., wherein the transformed-time varying wave is the carrier wave and is further modulated by an envelope [e.g., wherein the envelope is a sinusoidal wave; e.g., wherein the envelope has a monotonically increasing (in time) amplitude (e.g., wherein the envelope has a functional form corresponding to an increasing (in time) exponential)]; e.g., wherein the transformed time-varying wave is the envelope that modulates a carrier wave [e.g., wherein the carrier wave is a periodic wave (e.g., a sinusoidal wave; e.g., a square wave; e.g., a sawtooth wave)(e.g., having a higher frequency than the envelope)]}. In certain embodiments, a functional form of the waveform of the electronic drive signal is based on one or more recorded natural sounds (e.g., running water; e.g., ocean waves; e.g., purring; e.g., breathing; e.g., chanting; e.g., gongs; e.g., bells).

In certain embodiments, the method comprises receiving an electronic response signal from a monitoring device (e.g., directly from and/or to the monitoring device; e.g., via one or more intermediate server(s) and/or computing device(s))(e.g., a wearable monitoring device; e.g., a personal computing device; e.g., a fitness tracker;. e.g., a heart-rate monitor; e.g., an electrocardiograph (EKG) monitor; e.g., an electroencephalography (EEG) monitor; e.g., an accelerometer; e.g., a blood-pressure monitor; e.g., a galvanic skin response (GSR) monitor) and ), and wherein the controlling the waveform of the electronic drive signal comprises adjusting and/or selecting the waveform in response to (e.g., based on) the received electronic response signal.

In certain embodiments, the method comprises recording usage data (e.g., parameters such as a record of when the device was used, duration of use, etc.) and/or one or more biofeedback signals for a human subject [e.g., using one or more sensors, each operable to measure and record one or more biofeedback signals (e.g., a galvanic skin response (GSR) sensor; e.g., a heart-rate monitor; e.g., an accelerometer)] [e.g., storing and/or providing the recorded usage data and/or biofeedback signals for further processing and/or transmission to an external computing device, e.g., for computation (e.g., using a machine learning algorithm that receives the one or more biofeedback signals as input, along with, optionally, user reported information) and display of one or more performance metrics (e.g., a stress index) to a subject].

In certain embodiments, the method comprises automatically modulating and/or selecting the waveform of the electronic drive signal in response to (e.g., based on) the recorded usage data and/or biofeedback signals (e.g., using a machine learning algorithm that receives the one or more biofeedback signals as input, along with, optionally, user reported information, to optimize the waveform output).

In certain embodiments, a level [e.g., amplitude (e.g., a force; e.g., a displacement)] of at least a portion of the mechanical wave is (e.g., modulated and/or selected) based on activation thresholds of one or more target cells and/or proteins (e.g., mechanoreceptors (e.g., C tactile afferents); e.g., nerves; e.g., sensory thresholds corresponding to a level of tactile sensation) [e.g., wherein the one or more controller boards modulate the waveform output based on sub-activation thresholds (e.g., accounting for the response of the mechanical transducers)].

In certain embodiments, an amplitude of the mechanical wave corresponds to a displacement ranging from 1 micron to 10 millimeters (e.g., approximately 25 microns)(e.g., wherein the amplitude is adjustable over the displacement ranging from 1 micron to 10 millimeters)[e.g., wherein the amplitude corresponds to a force of approximately 0.4N][e.g., thereby matching the amplitude to activation thresholds of C tactile afferents].

In another aspect, the invention is directed to a method of treating anxiety and/or an anxiety related disorder in a subject by providing transcutaneous mechanical stimulation (e.g., non-invasive mechanical stimulation) to the subject via a stimulation device (e.g., a wearable device), the method comprising: generating a mechanical wave by a mechanical transducer of the stimulation device in response to an applied electronic drive signal; controlling a waveform of the electronic drive signal by a controller board (e.g., a controller board of the stimulation device; e.g., a remote controller board); and delivering the mechanical wave to a body location of the subject via the stimulation device, wherein the body location is in proximity to a temporal bone of the subject (e.g., wherein the temporal bone lies directly beneath the body location), thereby providing the transcutaneous mechanical stimulation to the subject.

In another aspect, the invention is directed to a method of treating anxiety and/or an anxiety related disorder in a subject by providing transcutaneous mechanical stimulation (e.g., non-invasive mechanical stimulation) to one or more nerves of the subject via a stimulation device (e.g., a wearable device), the method comprising: generating a mechanical wave by a mechanical transducer of the stimulation device in response to an applied electronic drive signal; controlling a waveform of the electronic drive signal by a controller board (e.g., of the stimulation device; e.g., a remote controller board); and delivering the mechanical wave to a body location of the subject via the wearable stimulation device, thereby stimulating the one or more nerves, wherein the one or more nerves comprise(s) a cranial nerve (e.g., vagus nerve; e.g., trigeminal nerve; e.g., facial nerve) of the subject.

In another aspect, the invention is directed to a method of treating anxiety and/or an anxiety related disorder in a subject by providing transcutaneous mechanical stimulation (e.g., non-invasive mechanical stimulation) to one or more nerves and/or mechanoreceptors of the subject via a stimulation device (e.g., a wearable device), the method comprising: generating a mechanical wave by a mechanical transducer of the stimulation device in response to an applied electronic drive signal; controlling a waveform of the electronic drive signal by a controller board (e.g., a controller board of the wearable stimulation device; e.g., a remote controller board), wherein the waveform comprises a frequency component ranging from approximately 5Hz to 15Hz (e.g., approximately 10 Hz; e.g., ranging from approximately 7 Hz to approximately 13 Hz; e.g., a frequency range matching an alpha brain wave frequency); and delivering the mechanical wave to a body location of the subject via the stimulation device, thereby providing the transcutaneous mechanical stimulation of the one or more nerves and/or mechanoreceptors of the subject.

In another aspect, the invention is directed to a method of treating anxiety and/or an anxiety related disorder in a subject by providing transcutaneous mechanical stimulation (e.g., non-invasive mechanical stimulation) to the subject via a stimulation device (e.g., a wearable device), the method comprising: generating a mechanical wave by a mechanical transducer of the stimulation device in response to an applied electronic drive signal; receiving an electronic response signal from a monitoring device (e.g., a wearable monitoring device) operable to monitor one or more physiological signals from the subject and generate, in response to the one or more physiological signals from the subject, the electronic response signal (e.g., wherein the electronic response signal is received directly from the monitoring device; e.g., wherein the electronic response signal is received from the wearable monitoring device via one or more intermediate servers and/or processors); responsive to the receiving the electronic response signal, controlling, via a controller board (e.g., a controller board of the stimulation device; e.g., a remote controller board), a waveform of the electronic drive signal to adjust and/or select the waveform based at least in part on the received electronic response signal; and delivering the mechanical wave to a body location of the subject via the stimulation device, thereby providing the transcutaneous mechanical stimulation to the subject.

In another aspect, the invention is directed to a method of treating anxiety and/or an anxiety related disorder in a subject by providing transcutaneous mechanical stimulation (e.g., non-invasive mechanical stimulation) to the subject via a stimulation device (e.g., a wearable device), the method comprising: (a) generating a mechanical wave by a mechanical transducer of the stimulation device in response to an applied electronic drive signal; (b) accessing and/or receiving [e.g., by a processor of a computing device, of and/or in communication with the stimulation device, e.g., an intermediate server and/or processor (e.g., of a mobile computing device in communication with the stimulation device)] subject response data (e.g., entered by the subjects themselves or biofeedback data recorded via sensors) and/or initialization setting data [e.g., physical characteristics of the subject (e.g., age, height, weight, gender, body-mass index (BMI), and the like); e.g., activity levels (e.g., physical activity levels); e.g., biofeedback data recorded by one or more sensors (e.g., included within the device and/or external to and in communication with the device)(e.g., a heart rate; e.g., a galvanic skin response; e.g., physical movement (e.g., recorded by an accelerometer)); e.g., results of a preliminary survey (e.g., entered by the subject themselves, e.g., via a mobile computing device, an app, and/or online portal; e.g., provided by a therapist/physician treating the subject for a disorder)]; (c) responsive to the accessed and/or received subject response data and/or initialization setting data, controlling, via a controller board (e.g., a controller board of the stimulation device; e.g., a remote controller board), a waveform of the electronic drive signal to adjust and/or select the waveform based at least in part on the subject response data and/or initialization setting data (e.g., using a machine learning algorithm that receives one or more biofeedback signals as input, along with, optionally, user reported information, to optimize the waveform output); and (d) delivering the mechanical wave to a body location of the subject via the stimulation device, thereby providing the transcutaneous mechanical stimulation to the subject.

In certain embodiments, step (b) comprises receiving and/or accessing subject response data [e.g., results of a survey recorded for the subject (e.g., entered by the subject themselves, e.g., via a mobile computing device, an app, and/or online portal; e.g., provided by a therapist/physician treating the subject for a disorder); e.g., biofeedback data recorded by one or more sensors (e.g., included within the device and/or external to and in communication with the device)(e.g., a heart rate; e.g., a galvanic skin response; e.g., physical movement (e.g., recorded by an accelerometer))] provided following their receipt of a round (e.g.,. a duration) of the transcutaneous mechanical stimulation provided by the stimulation device; and step (c) comprises controlling the waveform of the electronic drive signal based at least in part on the subject feedback, thereby modifying the transcutaneous mechanical stimulation provided to the subject based on subject response data.

In another aspect, the invention is directed to a method of treating anxiety and/or an anxiety related disorder in a subject by providing transcutaneous mechanical stimulation (e.g., non-invasive mechanical stimulation) to the subject via a stimulation device (e.g., a wearable device), the method comprising: generating a first mechanical wave by a first mechanical transducer of the stimulation device in response to a first applied electronic drive signal; controlling a first waveform of the first electronic drive signal by a controller board (e.g., a controller board of the stimulation device; e.g., a remote controller board); delivering the first mechanical wave to a first body location (e.g., on a right side; e.g., a location behind a right ear) of the subject via the stimulation device; generating a second mechanical wave by a second mechanical transducer of the stimulation device in response to a second applied electronic drive signal; controlling a second waveform of the second electronic drive signal by the controller board; and delivering the second mechanical wave to a second body location (e.g., on a left side; e.g., a location behind a left ear) of the subject via the stimulation device, thereby providing the transcutaneous mechanical stimulation to the subject.

In another aspect, the invention is directed to a method of treating anxiety and/or an anxiety related disorder in a subject by providing transcutaneous mechanical stimulation (e.g., non-invasive mechanical stimulation) to the subject via a stimulation device (e.g., a wearable device), the method comprising: generating a first mechanical wave by a first mechanical transducer of the stimulation device in response to an applied electronic drive signal; controlling a waveform of the first electronic drive signal by a controller board (e.g., a controller board of the stimulation device; e.g., a remote controller board); delivering the first mechanical wave to a first body location (e.g., on a right side; e.g., a location behind a right ear) of the subject via the stimulation device; generating a second mechanical wave by a second mechanical transducer of the stimulation device in response to the applied electronic drive signal; delivering the second mechanical wave to a second body location (e.g., on a left side; e.g., a location behind a left ear) of the subject via the stimulation device, thereby providing the transcutaneous mechanical stimulation to the subject.

In another aspect, the invention is directed to a method of treating anxiety and/or an anxiety related disorder in a subject by providing transcutaneous mechanical stimulation (e.g., non-invasive mechanical stimulation) to one or more nerves and/or mechanoreceptors of the subject via a stimulation device (e.g., a wearable device), in combination with one or more rounds of a therapy [e.g., psychotherapy; e.g., exposure therapy (e.g., for treatment of various phobias such as fear of heights, fear of public speaking, social phobia, panic attack, fear of flying, germ phobia, and the like); e.g., cognitive behavioral therapy (CBT); e.g., acceptance and commitment therapy (ACT)] the method comprising: generating a mechanical wave by a mechanical transducer of the stimulation device in response to an applied electronic drive signal;

controlling a waveform of the electronic drive signal by a controller board (e.g., a controller board of the wearable stimulation device; e.g., a remote controller board); and delivering the mechanical wave to a body location of the subject via the stimulation device at one or more times each in proximity to and/or during a round of the therapy received by the subject [e.g., prior to the round of therapy (e.g., such that the subject is in a more relaxed state prior to the round of the therapy; e.g., such that the subject is in a more responsive state prior to the round of the therapy; e.g., such that the subject is more open to an exposure; e.g., such that the subject is in a state of improved receptiveness and/or readiness to change); e.g., during the round of the therapy; e.g., following (e.g., immediately following) the round of the therapy; e.g., in between two or more rounds of therapy], thereby providing the transcutaneous mechanical stimulation of the one or more nerves and/or mechanoreceptors of the subject in combination with one or more rounds of the therapy.

In another aspect, the invention is directed to a method of treating anxiety and/or an anxiety related disorder in a subject by stimulating one or more nerves and/or mechanoreceptors of the subject (e.g., a human subject), the method comprising: using the device method comprising: using the device articulated in any of paragraphs [007] - [0043], for stimulation of the one or more nerves and/or mechanoreceptors of the subject.

In another aspect, the invention is directed to a method of treating anxiety and/or an anxiety related disorder in a human subject by stimulating one or more nerves of the human subject using a transcutaneous, neuromodulation device [e.g., a wearable device; e.g., a non-invasive device (e.g., not comprising any components that penetrate skin)], the device comprising one or more transducers (e.g., mechanical transducers), a battery, connectors, and one or more controller boards, wherein the one or more controller boards control waveform output through the connectors and the transducers, and wherein the transducers transcutaneously applied stimulates the one or more nerves, the method comprising: contacting the one or more transducers of the device to the human subject, generating the waveform output signal, activating the transducers using the waveform output signal (e.g., by applying the waveform output signal to the transducers to generate a mechanical wave), and stimulating the one or more nerves of the human subject, wherein the waveform output comprises an isochronic wave.

In another aspect, the invention is directed to a method of treating anxiety and/or an anxiety related disorder in a human subject by stimulating one or more mechanoreceptors of the human subject using transcutaneous stimulation device [e.g., a wearable device; e.g., a non-invasive device (e.g., not comprising any components that penetrate skin)], the device comprising one or more mechanical transducers, a battery, connectors, and one or more controller boards, wherein the one or more controller boards control waveform output through the connectors and the one or more mechanical transducers, and wherein the one or more mechanical transducers transcutaneously applied stimulate the one or more mechanoreceptors, the method comprising: contacting the one or more mechanical transducers of the device to the human subject, generating the waveform output signal, activating the mechanical transducers using the waveform output signal (e.g., by applying the waveform output signal to the transducers to generate a mechanical wave), and stimulating the one or more mechanoreceptors of the human subject, wherein the waveform output comprises an isochronic wave.

In another aspect, the invention is directed to a method of adjusting (e.g., controlling) a level of a stress hormone [e.g., cortisol (e.g., reducing a cortisol level); e.g., oxytocin (e.g., increasing an oxytocin level); e.g., serotonin (e.g., increasing a serotonin level)] in a subject, the method comprising transcutaneously delivering mechanical stimulation to the subject using a mechanical wave having a vibrational waveform selected to reduce the level of the stress hormone in the subject upon and/or following the delivering of the mechanical wave to the subject.

In another aspect, the invention is directed to a kit comprising the device of any one of the aspects and embodiments described herein and a label indicating that the device is to be used for reducing stress in a user as measured by a level of a stress hormone [e.g., cortisol (e.g., reducing a cortisol level); e.g., oxytocin (e.g., increasing an oxytocin level); e.g., serotonin (e.g., increasing a serotonin level)] for the subject.

In another aspect, the invention is directed to a transcutaneous neuromodulation device [e.g., a wearable device; e.g., a non-invasive device (e.g., not comprising any components that penetrate skin)] for treating a disorder in a subject (e.g., anxiety and/or an anxiety related disorder) by promoting nerve stimulation through mechanical vibration, comprising: one or more mechanical transducers, a battery, and a controller board, wherein the transducer, battery and controller board are in communication (e.g., through one or more connectors; e.g., wirelessly), and wherein the controller board controls waveform output through the transducer, thereby producing a mechanical vibration.

Elements of embodiments involving one aspect of the invention (e.g., compositions, e.g., systems, e.g., methods) can be applied in embodiments involving other aspects of the invention, and vice versa.

### BRIEF DESCRIPTION OF THE DRAWINGS

The foregoing and other objects, aspects, features, and advantages of the present disclosure will become more apparent and better understood by referring to the following description taken in conjunction with the accompanying drawings, in which:
**FIG.** 1 shows visual examples of some the devices used and their evolution.
**FIG. 2** shows visual examples of some of the actuator types and their placement.
**FIG. 3****.** shows examples of waveforms used in accordance with at least some embodiments of the present invention described herein.
**FIG. 4****.** shows summary of feasibility study associated with at least one of the embodiments disclosed herein.

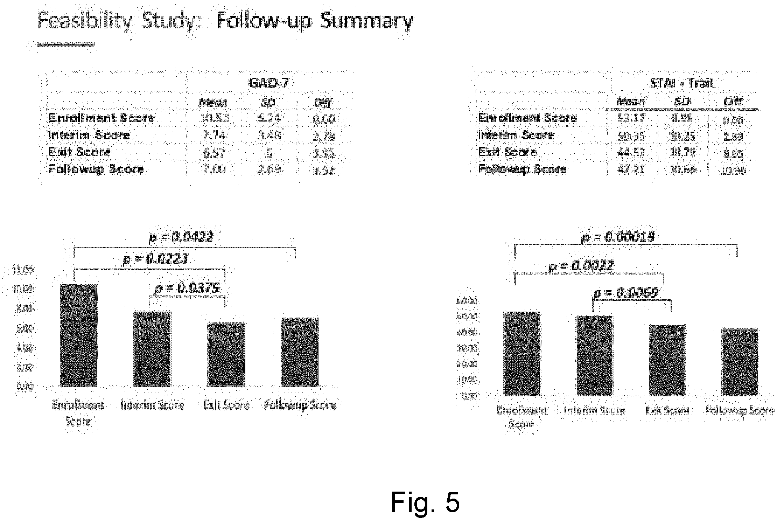

**FIG. 5** shows summary of follow up feasibility study associated with one of the embodiments disclosed herein.

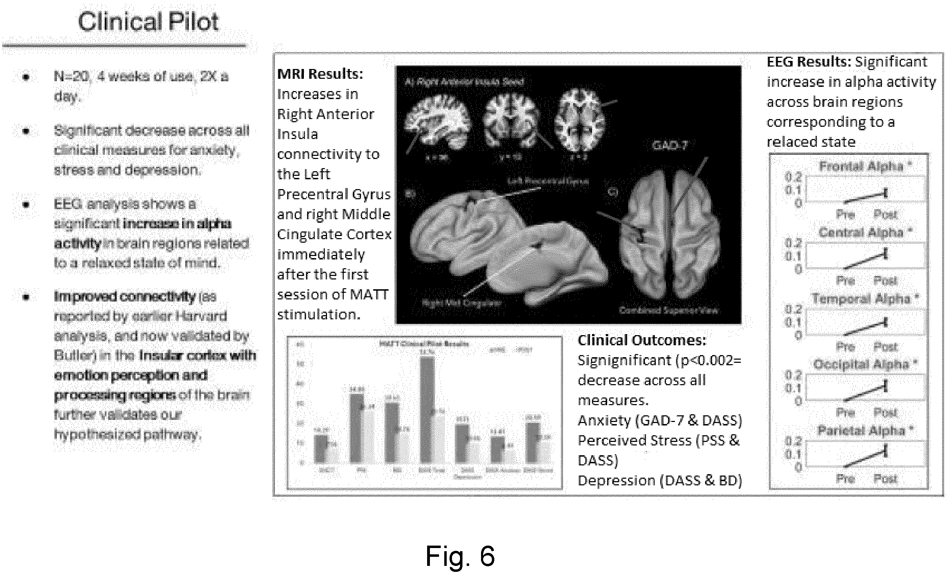

**FIG. 6** shows summary of clinical data associated with at least one embodiment disclosed herein.
**FIG. 7** shows a table of descriptive and clinical data for all participants.
**FIG. 8** shows table of data regarding pre-treatment functional connectivity relationships associated with post-MATT symptom improvement.
**FIG. 9** shows table of data regarding clusters associated with acute changes in resting-state functional connectivity after initial MATT administration.

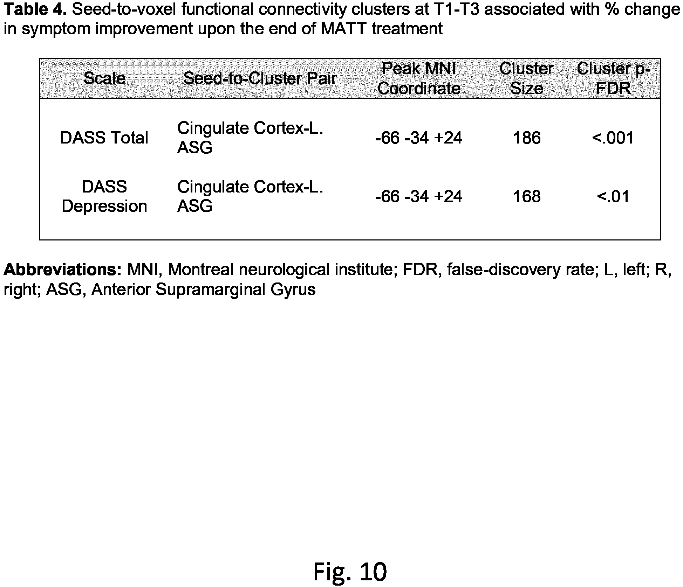

**FIG. 10** shows table of date regarding seed-to-voxel functional connectivity clusters at T1-T3 associated with % change in symptom improvement upon the end of MATT treatment.

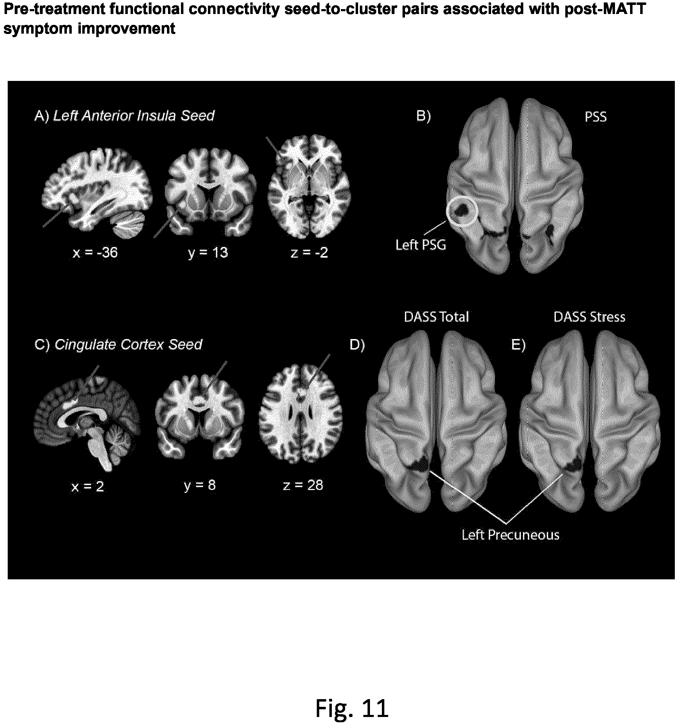

**FIG. 11** shows images of pre-treatment functional connectivity seed-to-cluster pairs associated with post-MATT symptom improvement. (A) Sagittal, Coronal, and Axial representations of the left anterior insula seed derived from Neurosynth; (B) Superior view of functional connectivity of the left anterior insula seed (as seen in A) to the left posterior supramarginal gyrus (PSG) cluster (circled in yellow) negatively correlated with post-treatment PSS scores (cross-validated p<.01); (C) Sagittal, Coronal, and Axial representations of the bilateral cingulate cortex seed derived from Neurosynth; (D) Superior view of functional connectivity of the cingulate cortex seed (as seen in C) to the left precuneus as associated with post-treatment improvement in total DASS scores (cross-validated p<.001); (E) Superior view of the same pattern of functional connectivity as seen in D, however this is associated with post-treatment improvement in DASS Stress scores (cross-validated p<.001). All neuroanatomical images were derived using CONN toolbox.

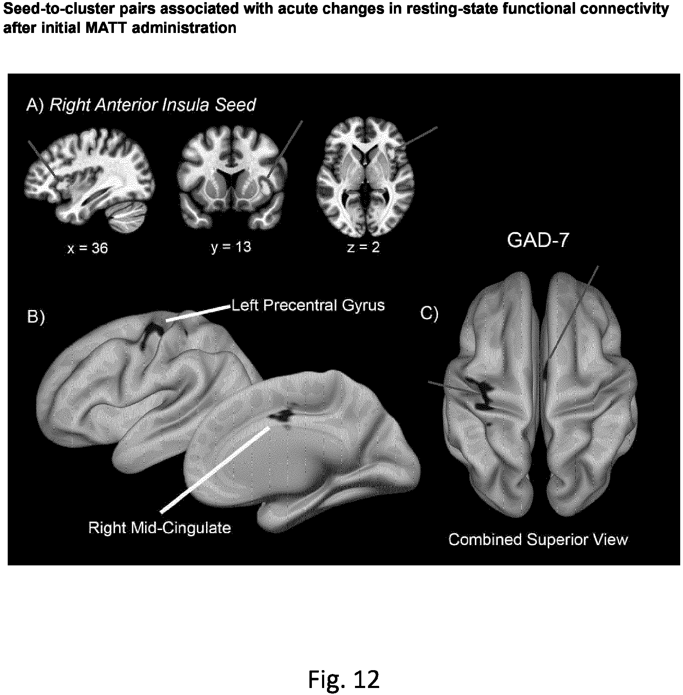

**FIG. 12** shows images of seed-to-cluster pairs associated with acute changes in resting-state functional connectivity after initial MATT administration. (A) Sagittal, Coronal, and Axial representations of the right anterior insula seed derived from Neurosynth; (B) Positive connectivity between the right anterior insula seed (as seen in A) and the left precentral gyrus (lateral view) and right mid-cingulate cortex (medial view) while controlling for baseline GAD-7 scores (cross-validated p<.001); (C) A combined superior view of the left precentral gyrus and right mid-cingulate cortex clusters while controlling for baseline GAD-7 scores. All neuroanatomical images were derived using CONN toolbox.

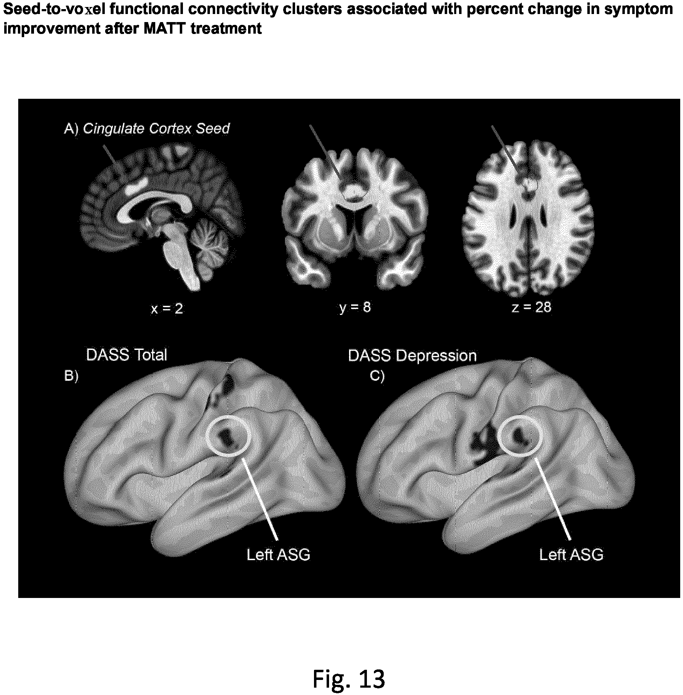

**FIG. 13** shows images of seed-to-voxel functional connectivity clusters associated with percent change in symptom improvement after MATT treatment. (A) Sagittal, Coronal, and Axial representations of the bilateral cingulate cortex seed derived from Neurosynth; (B) Lateral view of functional connectivity between the cingulate cortex seed (as seen in A) and the left anterior supramarginal gyrus (ASG) correlated with decreases in Total DASS scores after MATT completion (cross-validated p=.05); (C) Lateral view of the same pattern of functional connectivity as seen in B, however this is correlated with decreases in DASS Depression scores after MATT completion (cross-validated p=.06). All neuroanatomical images were derived using CONN toolbox.
**FIG. 14** shows supplementary table of descriptive data for participants grouped according to functional connectivity time point analyses (i.e. T1, T1-T2, and T1-T3).

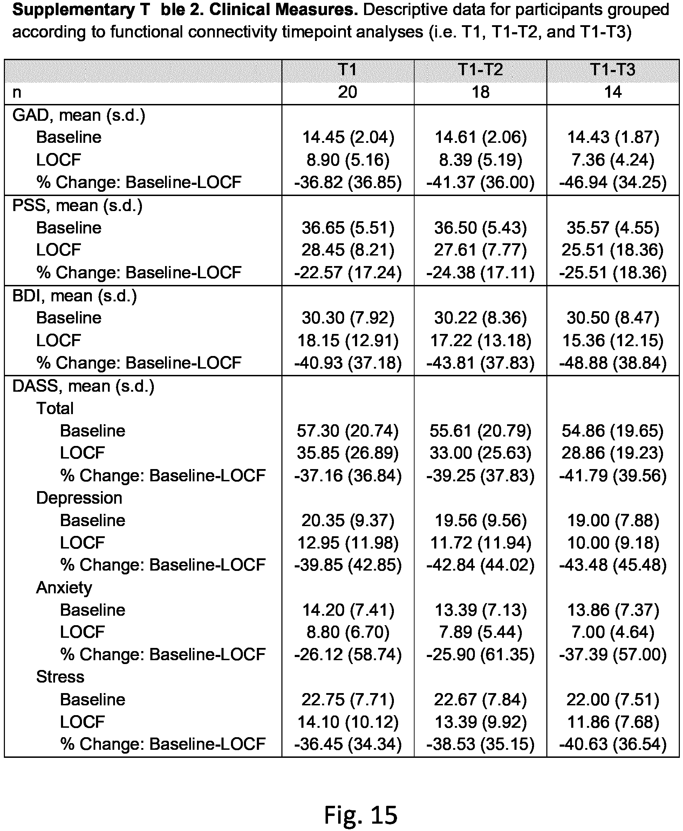

**FIG. 15** shows supplementary table of descriptive data for participants grouped according to functional connectivity time point analyses (i.e. T1, T1-T2, and T1-T3).

The features and advantages of the present disclosure will become more apparent from the detailed description set forth below when taken in conjunction with the drawings, in which like reference characters identify corresponding elements throughout. In the drawings, like reference numbers generally indicate identical, functionally similar, and/or structurally similar elements.

### DETAILED DESCRIPTION OF THE INVENTION

It is contemplated that systems, devices, methods, and processes of the claimed inventions described herein encompass variations and adaptations developed using information from the embodiments described herein. Adaptation and/or modification of the systems, architectures, devices, methods, and processes described herein may be performed, as contemplated by the embodiments described in this application and accompanying drawings, understanding that a person of ordinary skill in the art would know to make various modifications and adjustments to the embodiments described herein while still being covered by the claims of the present disclosure.

Throughout the description, where articles, devices, and systems are described as having, including, or comprising specific components, or where processes and methods are described as having, including, or comprising specific steps, it is contemplated that, additionally, there are articles, devices, and systems of the present invention that consist essentially of, or consist of, the recited components, and that there are processes and methods according to the present invention that consist essentially of, or consist of, the recited processing steps.

It should be understood that the order of steps or order for performing certain action is immaterial so long as the invention remains operable. Moreover, two or more steps or actions may be conducted simultaneously.

The mention herein of any publication, for example, in the Background section, is not an admission that the publication serves as prior art with respect to any of the claims presented herein. The Background section is presented for purposes of clarity and is not meant as a description of prior art with respect to any aspects of the current invention or any claim.

Anxiety disorders are the most prevalent mental health-related illnesses in the United States, affecting approximately 19.1% of adults annually [1] and 11.3% of Americans in their lifetime [1]. They are associated with severe social, occupational, and physical impairment [2, 3]; increased risk for chronic diseases including diabetes, cardiovascular disease, and asthma [1], and with engagement in maladaptive behaviors like smoking and heavy drinking [4, 5]. Anxiety disorders are also associated with greater use of disability days and decreased work productivity, placing a significant burden on the US economy and healthcare system [6].

Anxiety disorders are typically treated using a combination of psychotherapy and medication. Cognitive Behavioral Therapy (CBT) is the most frequently recommended form of psychotherapy [7]. In terms of pharmacotherapy, selective serotonin reuptake inhibitors (SSRIs) are favored over benzodiazepines [8, 9]. Combined CBT and SSRI therapy has proven clinical efficacy in treating panic disorder and generalized anxiety disorder [10, 11]. Though it is the gold standard treatment approach, this approach is not, however, universally effective. One-fifth of patients fail to complete treatment citing side effects, schedule/travel barriers, poor therapeutic alliances, and motivation as reasons for discontinuation [12]. Furthermore, even among those who complete a course of treatment, symptom improvement is inadequate in one-third of patients [12]. Given the substantial burden of under-treatment on patients and on society, there is a pressing need for novel anxiety disorder treatments.

Non-invasive peripheral nerve stimulation is one promising alternative treatment for anxiety disorders. During peripheral nerve stimulation, electrical or mechanical energy is delivered to the dermal area innervated by targeted nerves [13, 14]. Research has primarily focused on electrical stimulation methods (e.g., cranial, transcranial, and transcutaneous). Electrical stimulation reduces chronic lower back pain and acute post-surgical pain [15, 16]; initial findings indicate it also improves mood and anxiety disorder symptoms [17, 18]. Mechanical (acoustic) stimulation is comparatively understudied. Still, early studies have demonstrated ultrasound (> 20 KHz) stimulates Aβ peripheral nerves [19, 20], whereas low-frequency acoustic stimulation (< 20 KHz) of somatosensory mechanoreceptors enhances proprioception [21].

Mechanical Affective Touch Therapy (MATT) is a novel non-invasive peripheral mechanical nerve stimulation device for treatment of anxiety, stress and depression. The prototype of this wearable device resembles a commercially available MP3 player, but delivers gentle vibratory stimulation (via insulated transducers) to small areas of skin behind each ear on the temporal bone. The device is configured with an amplifier and piezoelectric elements or actuators (together, transducers) that enable a MP3-like signal generator to deliver gentle vibrations (< 20 Hz). Qualitatively, these vibrations resemble those from an electric toothbrush. It is hypothesized that higher level proprioception in response to vibratory stimulation occurs through Piezo2 ion channels during Merkel-cell mechano-transduction [22]. The resulting depolarizations subsequently drive peripheral Aβ and CT-afferent impulses to cortical somatosensory (S 1 and S2) and emotion processing regions (insula and anterior cingulate cortex) [23-25]. Thus, the MATT device ameliorates anxiety, depression and stress through targeted modulation of neural circuits involved in somato-sensation and pain.

Sham-controlled MATT studies indicate active transcutaneous vagus nerve stimulation (tVNS) increases magnetic resonance imaging (MRI) blood oxygen level-dependent (BOLD) activation in the insula, but BOLD decreases in the thalamus, posterior cingulate cortex, and parahippocampal gyrus [26]. These patterns have been replicated in multiple cohorts of healthy humans [27, 28]. In tVNS for depression, increases in functional connectivity between the precuneus, orbital prefrontal cortex, and select regions of the Default Mode Network (DMN) have been shown to correlate with reductions in depression symptoms [29].

The DMN is a functionally interconnected network of brain regions associated with introspection [30, 31], theory of mind [32], memory retrieval [33-35], and emotion regulation [36]. Major DMN regions include bilateral lateral and medial portions of the temporal and parietal cortex, the medial prefrontal cortex, hippocampus, and parahippocampus [37]. Clinically, the DMN is implicated in anxiety [38, 39] and mood disorders [40, 41]. For example, in anxious patients, DMN BOLD activation during emotion regulation is blunted compared to activation in healthy controls [42]. Studies measuring functional connectivity, a metric of functional cohesion between brain regions [43, 44], have also found evidence of more robust connectivity between the DMN and insula in patients with heightened anxiety [45]. As tVNS can modulate brain activity in both DMN and insula [26-29], - at least in healthy individuals - mechanical stimulation provides a treatment given the involvement of both in anxiety and pain [46, 47].

A study was done using resting-state functional connectivity (RSFC) to investigate the relationship between peripheral nerve stimulation for anxiety and cortical function. To our knowledge, this is the first study to examine non-invasive transcutaneous mechanical transduction's impact on brain connectivity. We evaluated the effects of MATT treatment on RSFC in pain and anxiety circuits in adults diagnosed with Axis I anxiety disorders participating in an open-label trial. We collected MRI data and standardized assessments of anxiety, depression, and stress across MATT's four-week course. RSFC data were collected: (1) before initial MATT stimulation (baseline), (2) immediately after baseline stimulation, and (3) after completion of the treatment course. We hypothesized that acute changes in connectivity and neural predictors of treatment response would localize to DMN. Moreover, we anticipated that changes in DMN connectivity would correspond to symptom changes across treatment.

### Methods and Study Overview

All participants received and used an active MATT research prototype device over 4 weeks. After the initial two MATT sessions (one at the MRI facility and the second in the research lab), participants were directed to self-administer MATT at home, or in another naturalistic setting, for at least two 20-minute sessions daily for 4 weeks. Resting-state functional MRI and structural MRI scans were obtained to enable investigation of brain changes associated with acute and chronic MATT. The first two MRI sessions occurred during the initial exposure to MATT stimulation. MRI data from time point one (T1) were acquired immediately before stimulation with MATT; time point two (T2) data were collected immediately after 15 minutes of MATT stimulation. We also collected resting state MRI data after completion of the 4-week MATT course (time point three; T3), which enabled us to evaluate brain correlates of symptom improvement after four weeks of MATT. Self-report scales assessing severity of anxiety, depression, and stress symptoms were collected at baseline, midpoint, and study endpoint.

### Participants

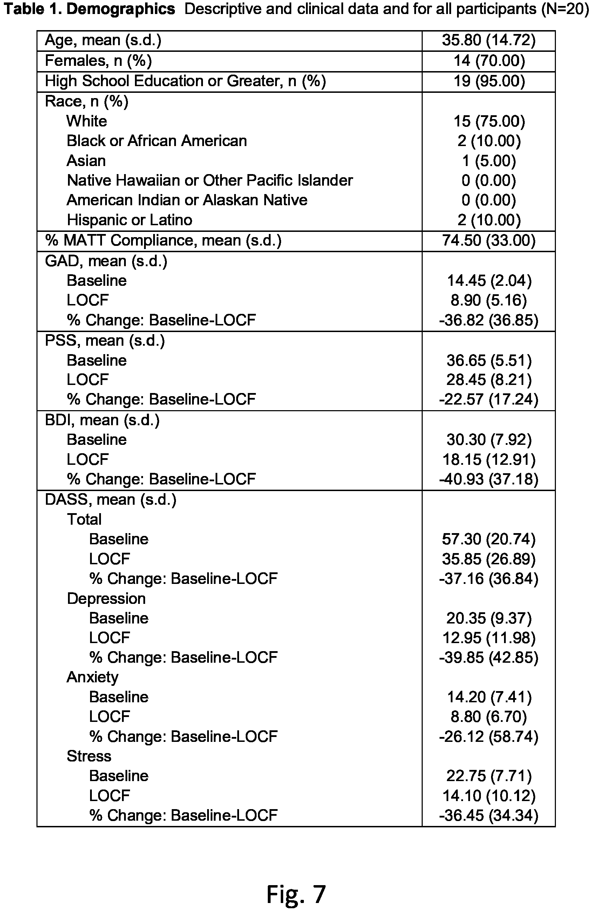

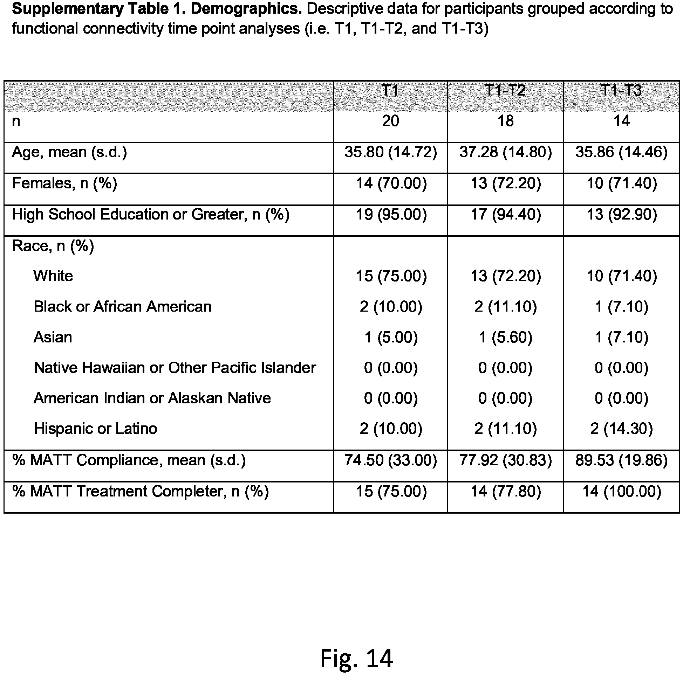

35 outpatients aged 18 to 65 years old with a current diagnosis of an Axis I anxiety disorder [48] were enrolled to participate in this study. Participants were recruited from the local and Butler Hospital community, obtaining written informed consent from all study participants. Participants were required to be medication-free or on a stable regimen of psychotropic medications (i.e., not started a new medication or changed doses of ongoing medications) for 30 days prior to the baseline visit and throughout the duration of their study participation. Participants were excluded if they had been psychiatrically hospitalized or had attempted suicide within the previous 6 months, had MRI safety contraindications, or were diagnosed with significant neurological conditions or another severe medical condition that could limit compliance with study procedures. All consent and study procedures were approved and supervised by the Butler Hospital Institutional Review Board. Twenty-one of the enrolled study participants completed the baseline MRI session and at least the midpoint study visits. 17 participants completed baseline and endpoint MRI scans. **FIGS. 7** and **14****,** including Table 1 Supplemental Table 1, show participant demographics and clinical assessment scores.

### Diagnostic Assessment and Medical Review

A trained clinical research assistant conducted the Mini-International Psychiatric Interview (MINI) [49] to confirm diagnosis of an Axis I Anxiety Disorder. Medical and neurological health histories and medication regimens for all participants were obtained and reviewed. As a part of this review, participants also completed a modified version of the Adverse Symptoms Checklist [50], a checklist used to monitor side effects in psychiatric clinical trials. Participants' scalps were visually inspected to confirm the absence of significant dermatological abrasion.

### Self-Report Questionnaires

Anxiety symptom severity was measured using the Generalized Anxiety Disorder 7 Item questionnaire (GAD-7) [51], which also served as the primary outcome measure. The participants' perception of stress was measured using the Perceived Stress Scale (PSS) [52]. To measure intensity of depression, participants were given the Beck Depression Inventory (BDI) [53]. Additionally, to measure negative emotional states of depression, anxiety, and stress, the Depression, Anxiety, and Stress Scale (DASS) [54] was administered. Individual scores for depression, anxiety, and stress were utilized, along with total composite scores.

### MATT Administration

The MATT device delivers gentle mechanical stimulation behind each ear via small (30 mm) piezoelectric disks which are mounted on a headset. The power and signal are generated from a modified MP3 player that effectively 'plays' the signal that the piezos convert to vibration. Individualized optimal stimulation was assigned by determining the sub-threshold vibrational level for each participant.

The first two stimulation sessions were administered by research staff concurrently with EEG collection or at their MRI visit and then participants were instructed to self-administer MATT at home or other naturalistic settings twice a day for four weeks. Once started, the device delivers stimulation for 20 minutes. The recommended trial dosing was two sessions a day with the option to use a third time if needed (i.e., if feeling more anxious or during anxiety-provoking situations). The MATT device used in this study, the piezos were driven by a sinusoidal 10 Hz signal that was delivered isochronically, having an active period of 2 seconds followed by 2 seconds of inactivity, called the 'refractory period'. The piezos vibrate with a displacement between .01 and .05 MM.

### MRI Data Collection and Preprocessing

All brain scan procedures took place at the Brown University MRI Facility. The first two scans occurred during Study Visit 2 (MRI baseline) and the final scans during Study Visit 6 (MRI endpoint) using a Siemens 3T MRI Scanner (Erlangen, Germany) and a 64-channel head coil. During Visit 2, prior to MATT stimulation, we collected a structural T1-weighted image (TE = 1.69 ms, TR = 2530 ms, FOV = 256 mm2, 1 mm3) and 10 minutes of resting-state functional MRI data (TE = 30 ms, TR = 1000 ms, FOV = 192 mm2, 2 mm3, 588 volumes). During the resting scan, participants were instructed to lay still and focus their gaze on a display screen showing a white crosshair in the middle of a black foreground. This screen was positioned at the back of the scanner bore and was viewed through a small MRI-safe mirror affixed to the scanner head coil. After these initial scans, the participant was removed from the scanner and underwent their first MATT stimulation session in a separate room. Immediately after, participants returned to the scanner for additional T1-weighted structural and resting state scans. Following completion of the last MATT session and final clinical assessments (Visit 5), additional structural and functional scans were collected. MATT was not administered during this MRI session.

All MRI data preprocessing steps were executed with the CONN Toolbox [55] (https://web.conn-toolbox.org). Standard MRI preprocessing steps included slice-time correction, motion estimation and realignment, normalization of images to Montreal Neurological Institute (MNI)-152 Atlas space, and spatial smoothing with a 6mm full-width half-max gaussian kernel. Additional functional connectivity preprocessing steps were applied to reduce the contribution of non-neuronal signals and motion on functional connectivity [56, 57]. We used the Anatomical CompCor method [58] to model non-neuronal signals: five principle components were computed from white matter and cerebrospinal fluid BOLD time-courses. These components were then regressed from subjects' preprocessed data along with six estimated motion parameters (3 translational, 3 rotational) and their first temporal derivatives, and the linear trend. Residuals were then band-pass filtered before first-level modeling.

### Subject-level Seed-To-Voxel Analyses

Functional regions-of-interest (ROI) or functional connectivity "seeds" were based on construct maps for "pain" and "anxiety" in Neurosynth (https://neurosynth.org/). Neurosynth [59] is a meta-analytic tool that generates functional connectivity maps for lexical terms and cognitive processes. To define our ROIs, each term's map using a minimum z-score and extracted clusters of spatially contiguous voxels were thresholded. Thresholding the "anxiety" map at z-scores >5 yielded two ROIs centered on the amygdala in each hemisphere. More stringent threshold (z-scores >7) for the "pain" ROIs to improve cluster separation were used. This produced bilateral clusters in the anterior insula and thalamus, and a mid-cingulate ROI crossing the sagittal midline.

For each seed, a whole-brain voxel-wise map of correlations with the seed's BOLD time-course was constructed. These subject-level maps underwent Fisher's R-to-Z transformation to improve conformation to the assumptions of generalized linear models. These seed maps were entered into second-level analyses of covariance (ANCOVA; see below) models for hypothesis testing.

### Second-Level Hypothesis Testing And Cross-Validation

Second-level models were constructed to: 1) identify pre-treatment connectivity patterns predictive of subsequent treatment outcomes; 2) localize acute connectivity changes immediately after MATT; 3) ask whether acute connectivity change predicts treatment outcome, and 4) identify post-treatment correlates of symptom improvement. All model results were evaluated using an uncorrected voxel-height threshold (p<.005) and were multiple comparisons corrected at p-FDR<.05. A leave-one-out cross-validation analysis was performed for all significant clusters. Briefly, on each iteration, models were re-estimated leaving one subject out and a parameter estimate (beta weight) was generated for the left-out subject based on this model. To determine if a cluster cross-validated, we submitted these estimated weights to a two-tailed t-test against the mean with alpha set at p<.05. We also excluded clusters if they were present in less than 80% of cross-validation masks. Only those results that survived leave-one-out cross-validation are presented in this disclosure.

To identify clusters predictive of treatment outcomes, continuous variables corresponding to clinical symptom scores were constructed at study endpoint (or last observation carried forward). Seed maps from the pre-treatment imaging session (timepoint one or T1) were entered into an ANCOVA model evaluating the between subjects' effect of endpoint scores after covariance for symptom severity at study baseline.

To localize acute effects of MATT, we compared pre-treatment (T1) seed maps to those collected immediately after MATT delivery (T2), evaluating within-subject change after covariance for baseline clinical symptoms. To localize brain regions where acute changes in functional connectivity were associated with subsequent improvement in clinical symptoms, we tested the between-subjects effect of post-treatment symptom change with session (T1>T2) as the within-subjects factor. Symptom change was operationalized as percent change in scale score (GAD, DASS, PSS, BDI) from baseline to endpoint, a procedure which normalizes baseline differences in symptom severity.

To identify functional correlates of clinical improvement, the significance of the between-subjects effect of symptom change on pre-treatment versus post-treatment (T1-T3) seed-to-voxel connectivity was tested.

### Morphometry Analyses

Freesurfer (v.5.3; http://surfer.nmr.mgh.harvard.edu/) software was used to explore the relationship between functional connectivity changes associated with MATT response and brain structure. Subjects' structural images from the T1 and T3 sessions were preprocessed using the 'fsrecon-all' routine. Steps included: skull stripping, volumetric labeling, intensity normalization, tissue parcellation, registration to Freesurfer's default spherical atlas ('fsaverage'), surface extraction, cortical labeling. For complete technical details of Freesurfer preprocessing, see [60-66]. We examined cortical thickness in the insula and mid-cingulate, as defined in [67], and subcortical volumes in MNI space in the left thalamus and amygdala. Metrics were calculated and extracted by Freesurfer. Volume estimates were adjusted to account for differences in brain volume. We then used SPSS (IBM; v25) to compute correlations between subject-level morphometry values and beta coefficients from the analysis of functional connectivity changes post-MATT. Statistical significance was evaluated at a one-tailed p<.05.

### Results

### Predictors Of Treatment Outcomes

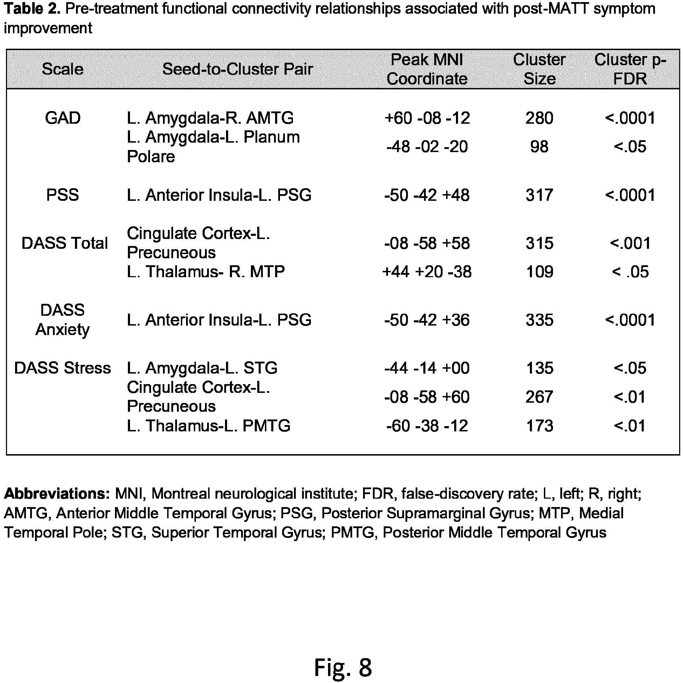

We found that stronger positive connectivity between pain and anxiety regions to the DMN, was generally predictive of greater clinical improvement at treatment endpoint. Functional connectivity of the left amygdala to DMN clusters in both the right and left lateral temporal cortex were negatively correlated with post-treatment GAD scores (both cross-validated p<.005). Similarly, functional connectivity of left anterior insula to left posterior supramarginal gyrus was also negatively correlated with post-treatment PSS scores (cross-validated p<.01; see Figure 1), though this part of parietal cortex is implicated in executive control networks, rather than DMN. Stronger positive connectivity between the cingulate pain ROI and the left precuneus (DMN) was also associated with post-treatment improvement in total DASS scores (cross-validated p<.001). Closer examination of DASS by subscale indicated that this relationship was driven by the stress dimension (See **FIG. 11****).** All findings remained significant after covariance for symptom severity at baseline and percent completed MATT sessions. See **FIG. 8****,** Table 2 for additional cluster information.

### Acute Changes In Functional Connectivity

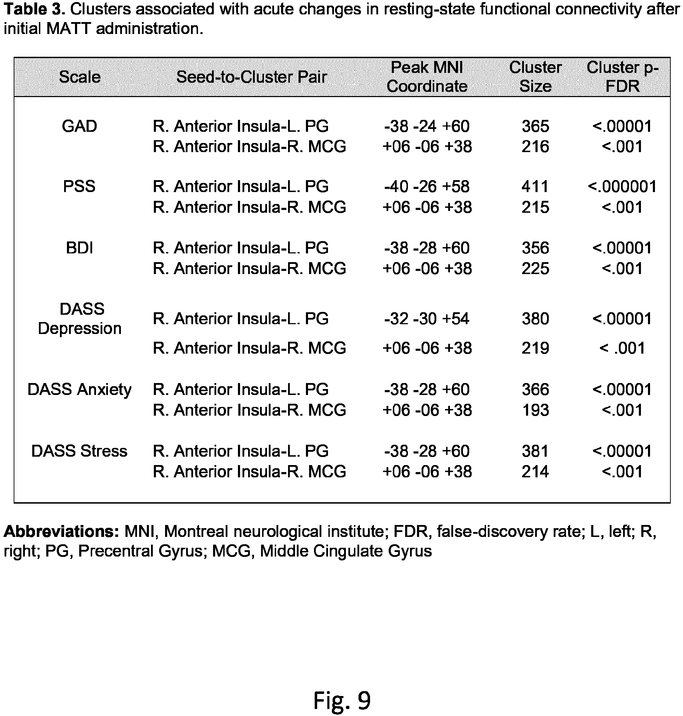

We observed increases in right anterior insula functional connectivity immediately after the initial session of MATT stimulation (See **FIG. 9** Table 3). Weak positive connectivity between the right insula seed and right middle cingulate cortex and left precentral gyrus observed at baseline became stronger after MATT (cross-validated p<.001; See **FIG. 12****).** Prior to stimulation, right insula and left precentral time courses were anti-correlated but became positively correlated after stimulation (cross-validated p<.005). Post hoc tests indicated that these connectivity relationships remained significant when baseline clinical symptoms were included as model covariates (GAD, BDI, PSS, DASS Total, DASS Stress, DASS Anxiety, and DASS Depression).

### Functional Connectivity And Post-Treatment Symptom Change

Increases in positive functional connectivity between the cingulate cortex and the left anterior supramarginal cortex after MATT completion were correlated with decreases in total DASS scores (cross-validated p=.05). Additional testing conducted within DASS subscales indicated that this connectivity relationship was associated with changes in depression (cross-validated p<.05) and stress (cross-validated p=.06), but not with changes in reported anxiety (see Table 4 and Figure 3).

### Exploratory Structure-Function Correlations

Our morphometry results preliminarily indicate that cortical thickness within pain circuits influences functional responsiveness to MATT. At baseline, greater cortical thickness in the right insula was associated with connectivity correlates of total DASS (r(14)=-0.40, p=.08) and DASS depression (r(14)=-0.48, p<.05) reductions post-MATT. Similar correlations were also observed in the left cingulate for total DASS (r(14)=-0.41, p=.07) and DASS depression (r(14)=-0.51, p<.05). We next computed percent change in cortical thickness across sessions T1 and T3 and correlated these against our connectivity change coefficients. While the relationship between changes in thickness and connectivity were marginally significant in the right insula (DASS total: r(14)=.42, p=.07; DASS depression: r(14)=.43, p=.06), correlations for the left cingulate were not significant (all p>.2).

The experiments described herein examined the relationship between changes in mood and anxiety symptoms and functional brain connectivity in individuals receiving peripheral nerve stimulation with the MATT device. The inventions described herein using MATT are the first acoustic, non-invasive mechanical stimulation device designed to treat anxiety, stress and depression. We also examined transcutaneous mechanical stimulation-induced changes in brain connectivity in patients with anxiety disorders. Broadly, our results indicate that MATT is capable of acute modulation of pain and anxiety networks and that modulation of connectivity between pain processing and internal mentation networks may be a key component of mechanical stimulation response.

As hypothesized, more robust pre-treatment functional connectivity between pain and anxiety regions, and the DMN predicted superior treatment response. Previous studies found that stronger connectivity between these networks is associated with anxiety severity [45, 68, 69]. In this study, we found that stronger inter-network connectivity predicted superior reduction in anxiety and stress symptoms. Specifically, stronger connectivity between the amygdala ("anxiety" seed) and the lateral temporal cortex at baseline was linked to greater anxiety reduction at the end of MATT. Similarly, stronger functional connectivity between the insula ("pain" seed) and the precuneus (DMN) was associated with larger decreases in stress. While DMN regions generally contribute to internally focused cognition, functional fractionations of this network link lateral temporal DMN to social cognition, and midsagittal DMN to affect and memory [70]. We speculate that stronger connectivity between anxiety regions and the DMN may facilitate safety learning through increased cross-network functional integration [71, 72].

In contrast to our a priori expectations, and observations from tVNS [26], we did not observe functional connectivity changes between pain and anxiety networks to DMN after initial MATT administration. Instead, we observed increases in insula connectivity to pain and motor regions including the mid-cingulate and postcentral cortex. Though the insula is associated with pain [73], it is also part of a broader network inclusive of midcingulate [74, 75] and postcentral cortex [76] involved in salience monitoring [77] and embodied sensation [78, 79]. We surmise that the observed pattern of acute connectivity increases could reflect the engagement of salience or haptic/pain monitoring in response to MATT stimulation.

Finally, we observed the anticipated correlation of changes in connectivity and symptoms post-treatment for pain seeds. Increases in mid-cingulate connectivity with the lateral subnetwork of the DMN were correlated with post-treatment reductions in depression and stress domain scores on the DASS. This prediction, however, did not hold for our anxiety seeds. This null finding may reflect our use of a naturalistic sample and stricter inclusion criteria for anxiety versus other clinical symptoms. Alternatively, our results may indicate that the latency to response differs between clinical symptoms or that MATT modifies different networks across the treatment course. To wit, we observed changes in pain network connectivity to salience and interoception regions acutely, whereas post-treatment pain connectivity effects localized to DMN. Our preliminary structural results also highlight the centrality of pain and salience circuits to MATT response.

### Limitations

Several limitations of the experiments disclosed herein must be noted. First, this preliminary study used an open-label design without a sham control condition. Though promising, it remains to be seen if results will replicate in a blinded, randomized control trial (RCT). We also note that our sample size was small, and despite cross-validations, these imaging results should be regarded as preliminary until replicated by a larger sample RCT. In addition, our primary measure of anxiety, the GAD-7, had fewer questions and thus less variability than our measures of depression and stress, potentially leading to more non-significant findings in relation to anxiety symptoms. Finally, though we speculate that our findings are suggestive of an underlying temporal heterogeneity in the response of brain networks to MATT, we acknowledge that the evaluation of functional connectivity at rest, rather than on task may introduce network bias.

### Conclusion

In summary, MATT is a novel treatment to alleviate and reduced anxiety, stress and depression in a human after altering resting state functional connectivity in the DMN after both acute and long-term administration. Analyses revealed that MATT-induced increased connectivity between pain and anxiety ROIs and the posterior DMN correlate with decreases in anxiety, stress and depression. This study is an important first step in developing non-invasive alternative anxiety treatments that alleviate symptoms through alteration of brain connectivity.

### References

1. Strine, T.W., et al., Depression and Anxiety in the United States: Findings From the 2006 Behavioral Risk Factor Surveillance System. Psychiatric Services, 2015. 59(12): p. 1383-1390.
2. Ramsawh, H.J., et al., Relationship of anxiety disorders, sleep quality, and functional impairment in a community sample. Journal of Psychiatric Research, 2009. 43(10): p. 926-933.
3. Kessler, R.C., et al., Prevalence, severity, and comorbidity of 12-month DSM-IV disorders in the National Comorbidity Survey Replication. Arch Gen Psychiatry, 2005. 62(6): p. 617-27.
4. Fluharty, M., et al., The Association of Cigarette Smoking With Depression and Anxiety: A Systematic Review. Nicotine Tob Res, 2017. 19(1): p. 3-13.
5. Novak, A., et al., Anxiety sensitivity, self-reported motives for alcohol and nicotine use, and level of consumption. Journal of Anxiety Disorders, 2003. 17(2): p. 165-180.
6. Wittchen, H.U., Generalized Anxiety Disorder: Prevalence, Burden, and Cost to Society. Depression and Anxiety, 2002. 16(4): p. 162-171.
7. Kaczkurikin, A.N., Cognitive-behavioral therapy for anxiety disorders: an update on the empirical evidence. Dialogues in Clinical Neuroscience, 2015. 17(3): p. 337-346.
8. Parsailk, A.K., et al., Mortality associated with anxiolytic and hypnotic drugs-A systematic review and metanalysis. Aust N Z J Psychiatry, 2016. 50(6): p. 520-533.
9. Thibaut, T., Anxiety disorders: a review of current literature. Dialogues in Clinical Neuroscience, 2017. 19(2): p. 87-88.
10. Cuijpers, P., et al., Adding Psychotherapy to Antidepressant Medication in Depression and Anxiety Disorders: a Meta-Analysis. World Psychiatry, 2014. 13: p. 56-67.
11. Hofmann, S.G., et al., Is it Beneficial to Add Pharmacotherapy to Cognitive-Behavioral Therapy When Treating Anxiety Disorders? A Meta-Analytic Review. International Journal of Cognitive Therapy, 2009. 2: p. 160-175.
12. Taylor, S., J.S. Abramowitz, and D. McKay, Non-adherence and Non-Response in the Treatment of Anxiety Disorders. J Anxiety Disord, 2012. 26(5): p. 583-589.
13. Beekwilder, J.P. and T. Beems, Overview of the clinical applications of vagus nerve stimulation. Journal of Clinical Neurophysiology, 2010. 27(2): p. 130-138.
14. Gibson, T.H. and D.E. O'Hair, Cranial application of low-level transcranial electrotherapy vs. relaxation instruction in anxious patients. American Journal of Electromedicine, 1987. 4(1): p. 18-21.
15. Gozani, S.N., Fixed-site high-frequency transcutaneous electrical nerve stimulation for treatment of chronic low back and lower extremity pain. J Pain Res, 2016. 9: p. 469-79.
16. DeSantana, J.M., et al., Effectiveness of transcutaneous electrical nerve stimulation for treatment of hyperalgesia and pain. Curr Rheumatol Rep, 2008. 10(6): p. 492-9.
17. Hein, E., et al., Auricular transcutaneous electrical nerve stimulation in depressed patients: a randomized controlled pilot study. J Neural Transm (Vienna), 2013. 120(5): p. 821-7.
18. De Felice, E.A., Cranial Electrotherapy Stimulation (CES) in the Treatment of Anxiety and Other Stress-Related Disorders: A Review of Controlled Clinical Trials. Stress Medicine, 1999. 13(1): p. 31-42.
19. Legon, W., et al., Pulsed Ultrasound Differentially Stimulates Somatosensory Circuits in Humans as Indicated by EEG and fMRI. Plos One, 2012. 7(12): p. e51177.
20. Gavrilov, L.R., et al., The effect of focused ultrasound on the skin and deep nerve structures of man and animal. Prog Brain Res, 1976. 43: p. 279-92.
21. Priplata, A.A., et al., Vibrating insoles and balance control in elderly people. Lancet, 2003. 362(9390): p. 1123-4.
22. Woo, S.H., et al., Piezo2 is required for Merkel-cell mechanotransduction. Nature, 2014. 509: p. 622-626.
23. Ikeda, R., et al., Merkel cells transduce and encode tactile stimuli to drive Abeta-afferent impulses. Cell, 2014. 157(3): p. 664-75.
24. Olausson, H., et al., The neurophysiology of unmyelinated tactile afferents. Neurosci Biobehav Rev, 2010. 34(2): p. 185-91.
25. Zaki, J., et al., Different circuits for different pain: Patterns of functional connectivity reveal distinct networks for processing pain in self and others. Soc Neurosci, 2007. 2(3-4): p. 276-91.
26. Kraus, T., et al., CSN BOLD fMRI Effects of Sham-Controlled Transcutaneous Electrical Nerve Stimulation in the Left Outer Auditory Canal- A Pilot Study. Brain Stimulation, 2013. 6(5): p. 798-804.
27. Frangos, E. and B.R. Komisaruk, Access to Vagal Projections via Cutaneous Electrical Stimulation of the Neck: fMRI Evidence in Healthy Humans. Brain Stimul, 2017. 10(1): p. 19-27.
28. Kraus, T., et al., BOLD fMRI deactivation of limbic and temporal brain structures and mood enhancing effect by transcutaneous vagus nerve stimulation. J Neural Transm (Vienna), 2007. 114(11): p. 1485-93.
29. Fang, J., et al., Transcutaneous Vagus Nerve Stimulation Modulates Default Mode Network in Major Depressive Disorder. Biol Psychiatry, 2016. 79(4): p. 266-73.
30. Otti, A., et al., I Know the Pain You Feel-How the Human Brain's Default Mode Predicts Our Resonance to Another's Suffering. Neuroscience, 2010. 169(1): p. 143-148.
31. Crone, J.S., et al., Deactivaion of the Default Mode Network as a Marker of Impaired Consciousness: An fMRI Study. PLOS ONE, 2011. 6(10): p. e26373.
32. Spreng, R.N. and C.L. Grady, Patterns of Brain Activity Supporting Autobiographic Memory, Prospection, and Theory of Mind, and Their Relationship to the Default Mode Network. Journal of Cognitive Neuroscience, 2010. 22(6): p. 1112-23.
33. Kim, H., Dissociating the roles of the default-mode, dorsal, and ventral networks in episodic memory retrieval. Neuroimage, 2010. 50(4): p. 1648-57.
34. Wirth, M., et al., Semantic memory involvement in the default mode network: A functional neuroimaging study using independent component analysis. Neuroimage, 2011. 54(4): p. 3057-3066.
35. Philippi, C.L., et al., Damage to the default mode network disrupts autobiographical memory retrieval. Soc Cogn Affect Neurosci, 2015. 10(3): p. 318-26.
36. Pan, J.H., et al., Emotion Regulation and Complex Brain Networks: Association Between Expressive Suppression and Efficiency in the Fronto-Parietal Network and Default-Mode Network. Frontiers in Human Neuroscience, 2018. 12(70).
37. Raichle, M.E., The Brain's Default Mode Network. Annu Rev Neurosci, 2015. 38: p. 433-447.
38. Zhao, X.H., et al., Altered default mode network activity in patient with anxiety disorders: an fMRI study. Eur J Radiol, 2007. 63(3): p. 373-8.
39. Tao, Y., et al., The Structural Connectivity Pattern of the Default Mode Network and Its Association with Memory and Anxiety. Front Neuroanat, 2015. 9: p. 152.
40. Sheline, Y.I., et al., The default mode network and self-referential processes in depression. Proc Natl Acad Sci USA, 2009. 106(6): p. 1942-7.
41. Wise, T., et al., Instability of default mode network connectivity in major depression: a two-sample confirmation study. Transl Psychiatry, 2017. 7(4): p. e1105.
42. Sylvester, C.M., et al., Functional network dysfunction in anxiety and anxiety disorders. Trends Neurosci, 2012. 35(9): p. 527-35.
43. Lowe, M.J., et al., Correlations in low-frequency BOLD fluctuations reflect cortico-cortical connections. Neuroimage, 2000. 12(5): p. 582-7.
44. van den Heuvel, M.P. and P. Hulshoff, Exploring the brain network: A review on resting-state fMRI functional connectivity. European Neuropsychopharmacology, 2010. 20(8): p. 519-534.
45. Dennis, E.L., et al., Resting-State Functional Magnetic Resonance Imaging in Youth and Adults. Brain Connectivity, 2011. 1(3): p. 245-254.
46. Zhang, S., et al., Resting-state connectivity in the default mode network and insula during experimental low back pain. Neural Regen Res, 2014. 9(2): p. 135-142.
47. Kuci, A., et al., Enhanced Medial Prefrontal-Default Mode Network Functional Connectivity in Chronic Pain and Its Association with Pain Rumination. Journal of Neuroscience, 2014. 34(11): p. 3969-3975.
48. World Health Organization. The ICD-10 classification of mental and behavioural disorders: clinical descriptions and diagnostic guidelines. Geneva: World Health Organization; 1992.
49. Sheehan, D.V., Lecrubier, Y., Sheehan, K.H., et al. The Mini-International Neuropsychiatric Interview (M.I.N.I.): the development and validation of a structured diagnostic psychiatric interview for DSM-IV and ICD-10. J Clin Psychiatry, 1998. 59(Suppl 20): p. 22-57.
50. Levine, J. and N.R. Schooler, SAFETEE: a technique for the systematic assessment of side effectsin clinical trials. Psychopharmacol Bull., 1986. 22(2): p. 343-81.
51. Spitzer R.L., Kroenke K., Williams J.B.W., Löwe B. A Brief Measure for Assessing Generalized Anxiety Disorder: The GAD-7. Arch Intern Med., 2006. 166(10): p. 1092-1097.
52. Cohen, S., Kamarck, T., Mermelstein, R. A global measure of perceived stress. Journal of health and social behavior, 1983; 385-396.
53. Beck, A.T., et al. An inventory for measuring depression. Archives of general psychiatry, 1961. 4(6): p. 561-71.
54. Parkitny L., McAuley J. The Depression Anxiety Stress Scale (DASS). J Physiother., 2010. 6(3): p. 204.
55. Whitfield-Gabrieli, S. and A. Nieto-Castanon, Conn: a functional connectivity toolbox for correlated and anticorrelated brain networks. Brain Connect, 2012. 2(3): p. 125-41.
56. Power, J.D., et al., Spurious but systematic correlations in functional connectivity MRI networks arise from subject motion. Neuroimage, 2012. 59(3): p. 2142-54.
57. Ciric, R., et al., Benchmarking of participant-level confound regression strategies for the control of motion artifact in studies of functional connectivity. Neuroimage, 2017.
58. Behzadi, Y., et al., A component based noise correction method (CompCor) for BOLD and perfusion based fMRI. Neuroimage, 2007. 37(1): p. 90-101.
59. Yarkoni, T., et al., Large-scale automated synthesis of human functional neuroimaging data. Nature methods, 2011. 8(8): p. 665.
60. Dale, A.M., Fischl, B., Sereno, M.I. Cortical surface-based analysis. I. Segmentation and surface reconstruction. Neuroimage, 1999. 9: p. 179-194.
61. Fischl, B., Dale, A.M., Measuring the thickness of the human cerebral cortex from magnetic resonance images. Proc Natl Acad Sci USA, 2000. 97: p. 11050-11055.
62. Fischl, B., Salat, D.H., Busa, E., Albert, M., Dieterich, M., Haselgrove, C., van der Kouwe, A., Killiany, R., Kennedy, D., Klaveness, S., Montillo, A., Makris, N., Rosen, B., Dale, A.M. Whole brain segmentation: automated labeling of neuroanatomical structures in the human brain. Neuron, 2002. 33: p. 341-355.
63. Fischl, B., Sereno, M.I., Dale, A.M. Cortical surface-based analysis. II: Inflation, flattening, and a surface-based coordinate system. Neuroimage, 1999a. 9: p. 195-207.
64. Fischl, B., Sereno, M.I., Tootell, R.B., Dale, A.M. High-resolution intersubject averaging and a coordinate system for the cortical surface. Hum Brain Mapp, 1999b. 8: p. 272-284.
65. Fischl, B., van der Kouwe, A., Destrieux, C., Halgren, E., Segonne, F., Salat, D.H., Busa, E., Seidman, L.J., Goldstein, J., Kennedy, D., Caviness, V., Makris, N., Rosen, B., Dale, A.M. Automatically parcellating the human cerebral cortex. Cereb Cortex, 2004b.14: p. 11-22.
66. Reuter, M., Rosas, H.D., Fischl, B. Highly Accurate Inverse Consistent Registration: A Robust Approach. Neuroimage, 2010. 53(4): p. 1181-1196.
67. Desikan, R.S., Ségonne, F., Fischl, B., et al. An automated labeling system for subdividing the human cerebral cortex on MRI scans into gyral based regions of interest. Neuroimage, 2006. 31(3): p. 968-980.
68. Liao, W., et al., Selective abberant functional connectivity of resting state networks in social anxiety disorder. Neuroimage, 2010. 52(4): p. 1549-1558.
69. Lanius, R.A., et al., Default mode network connectivity as a predictor of post-traumatic stress disorder symptom severity in acutely traumatized subjects. Acta Psychiatrica Scandinavica 2009. 121(1): p. 33-40.
70. Andrews-Hanna, J.R., et al., Functional-anatomic fractionation of the brain's default network. Neuron, 2010. 65(4): p. 550-62.
71. Laing, P.A.F., N. Burns, and I. Baetu, Individual differences in anxiety and fear learning: The role of working memory capacity. Acta Psychol (Amst), 2019. 193: p. 42-54.
72. Nakajima, M., K. Takano, and Y. Tanno, Adaptive functions of self-focused attention: Insight and depressive and anxiety symptoms. Psychiatry Res, 2017. 249: p. 275-280.
73. Lu, C.B., et al., Insular Cortex is Critical for the Perception, Modulation, and Chronification of Pain. Neuroscience Bulletin, 2016. 32(2): p. 191-201.
74. Uddin, L.Q., Salience processing and insular cortical function and dysfunction. Nat Rev Neurosci, 2015. 16(1): p. 55-61.
75. Wiech, K., et al., Anterior Insula Integrates Information about Salience into Perceptual Decisions about Pain. Journal of Neuroscience, 2010. 30(48): p. 16324-16331.
76. Ellard, K.K., et al., Functional Connectivity Between Anterior Insula and Key Notes of Frontoparietal Executive Control and Salience Networks Distinguish Bipolar Depression from Unipolar Depression and Healthy Control Subjects. Biological Psychiatry: Cognitive Neuroscience and Neuroimaging, 2018. 3(5): p. 473-484.
77. Menon, V. and L.Q. Uddin, Saliency, switching, attention and control: a network model of insula function. Brain Struct Funct, 2010. 214(5-6): p. 655-67.
78. Allen, M., et al., Anterior insula coordinates hierarchical processing of tactile mismatch responses. Neuroimage, 2016. 127: p. 34-43.
79. Gallese, V., Embodied simulation: From neurons to phenomenal experience. Phenomenology and the Cognitive Sciences, 2005. 4: p. 23-48.

## Claims

1. A device for reducing anxiety in a human, the device comprising:
one or more mechanical transducers,
one or more batteries,
one or more sinusoidal waveforms and
one or more controller boards that control at least the one or more sinusoidal waveforms output through the mechanical transducers;
wherein the one or more mechanical transducers, the one or more batteries and the one or more controller boards are in communication;
wherein the controller board controls sinusoidal waveform output through the one or more mechanical transducers, thereby producing mechanical vibrations for a human and wherein when the device is adapted to provide mechanical vibrations in proximity to the temporal bone of the human's head.

2. The device of claim 1, wherein the frequency of the one or more waveform is less than 20 Hz.

3. The device of claim 1, wherein the frequency of the one or more waveforms is approximately 10 Hz.

4. The device of claims 1, wherein the one or more waveforms are isochronic.

5. A device for reducing depression in a human, the device comprising:
one or more mechanical transducers,
one or more batteries, and
one or more sinusoidal waveforms and
one or more controller boards that control at least the one or more sinusoidal waveforms output through the mechanical transducers;
wherein the one or more mechanical transducers, the one or more batteries and the one or more controller boards are in communication;
wherein the controller board controls sinusoidal waveform output through the one or more mechanical transducers, thereby producing mechanical vibrations for a human and wherein when the device is adapted to provide mechanical vibrations in proximity to the temporal bone of the human's head.

6. The device of claim 5, wherein the frequency of the one or more waveform is less than 20 Hz.

7. The device of claim 5, wherein the frequency of the one or more waveforms is approximately 10 Hz.

8. The device of claims 5, wherein the one or more waveforms are isochronic.

9. A device for reducing stress in a human, the device comprising:
one or more mechanical transducers,
one or more batteries, and
one or more sinusoidal waveforms and
one or more controller boards that control at least the one or more sinusoidal waveforms output through the mechanical transducers;
wherein the one or more mechanical transducers, the one or more batteries and the one or more controller boards are in communication;
wherein the controller board controls sinusoidal waveform output through the one or more mechanical transducers, thereby producing mechanical vibrations for a human and wherein when the device is adapted to provide mechanical vibrations in proximity to the temporal bone of the human's head.

10. The device of claim 9, wherein the frequency of the one or more waveform is less than 20 Hz.

11. The device of claim 9, wherein the frequency of the one or more waveforms is approximately 10 Hz.

12. The device of claim 9, wherein the one or more waveforms are isochronic.

13. A method of reducing anxiety, stress or depression in a human, the method comprising:
generating mechanical vibrations using a sinusoidal waveform and a mechanical transducer of a transcutaneous mechanical stimulation device in response to an applied electronic drive signal;
controlling the mechanical vibrations of the electronic drive signal by a controller board so that the mechanical vibrations have a frequency of less than 20 Hz; and
delivering the mechanical vibrations to the body of the human via the mechanical stimulation device, thereby providing the human with transcutaneous mechanical stimulation that reduces the human's anxiety, stress and/or depression.

14. The method of claim 13, wherein the frequency of the one or more waveforms is approximately 10 Hz.

15. The method of claim 13, wherein the one or more waveforms are isochronic.
